# NEUE EUROPÄISCHE PATENTSCHRIFT

(11) **EP 2 996 670 B2**
(45) Veröffentlichungstag und Bekanntmachung des Hinweises auf die Entscheidung über den Einspruch: **18.11.2026**
(45) Hinweis auf die Patenterteilung: 18.07.2018
(21) Anmeldenummer: 14705993.5
(22) Anmeldetag: 11.02.2014
(51) Int. Cl.: A61K 8/45, A61Q 5/08, A61K 8/65, A61K 8/73, A61K 8/891, A61K 8/22, A61K 8/23, A61K 8/898

(54) **TRANSPARENTE BLONDIERMITTEL MIT PROTEINEN UND/ODER SILIKONÖLEN**
TRANSPARENT BLEACHING COMPOSITIONS WITH PROTEINS AND/OR SILICONE OILS
PRODUIT DE DÉCOLORATION TRANSPARENT AVEC PROTÉINES ET/OU HUILES DE SILICONE

(30) Priorität: 16.05.2013 DE 102013209098
(43) Veröffentlichungstag der Anmeldung: 23.03.2016
(73) Patentinhaber: Henkel AG & Co. KGaA, 40589 Düsseldorf (DE)
(72) Erfinder: ANDERHEGGEN, Bernd, 41189 Mönchengladbach (DE); JANSSEN, Frank, 51103 Köln (DE)
(74) Vertreter: LKGLOBAL Partnerschaftsgesellschaft mbB
(86) Internationale Anmeldenummer: PCT/EP2014/052590
(87) Internationale Veröffentlichungsnummer: WO 2014/183882

(56) Entgegenhaltungen:
- DE-A1- 10 315 421
- DE-A1- 102004 061 468
- DE-A1- 102006 008 149
- DE-A1- 102010 030 337
- JP-A- 2001 247 437
- US-A1- 2006 210 499
- US-A1- 2011 126 361
- US-A1- 2012 315 236

## Beschreibung

Die vorliegende Erfindung betrifft Mittel zur oxidativen Farbveränderung im Bereich der Kosmetik, die sich besonders zum Aufhellen keratinischer Fasern, insbesondere menschlicher Haare, eignen. Die in Blondiermitteln enthaltenen Oxidationsmittel sind in der Lage, die Haarfaser durch die oxidative Zerstörung des haareigenen Farbstoffes Melanin aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein, für das Erzielen eines stärkeren Blondiereffektes wird üblicherweise eine Mischung aus Wasserstoffperoxid und Peroxodisulfatsalzen und/oder Peroxomonosulfatsalzen eingesetzt.

Aus Stabilitätsgründen werden handelsübliche Blondiermittel gewöhnlich in zwei getrennt voneinander verpackten Zubereitungen angeboten, die unmittelbar vor der Anwendung zu einer fertigen Anwendungszubereitung vermischt werden. Üblicherweise bestehen handelsübliche Blondiermittel aus einer flüssigen Oxidationsmittelzubereitung und einem Pulver, das feste Oxidationsmittel enthält. Produkte mit weiteren Komponenten werden ebenfalls im Handel angeboten.

Einen wichtigen Aspekt der Anwendung von Blondiermitteln stellt die Kontrolle des Aufhellvorgangs auf der Faser dar. Üblicherweise wird der Entfärbevorgang mindestens ein Mal während der Einwirkzeit vom Friseur kontrolliert. Handelsübliche Blondiermittel sind in anwendungsbereitem Zustand in der Regel weiße bis farbige, trübe Gele oder Emulsionen. Bei der Anwendung dieser Blondiermittel ist es zur Kontrolle des Entfärbevorgangs während der Einwirkzeit notwendig, das Mittel in einem oder mehreren Bereichen der Fasern abzutragen. Der Fortschritt der Farbveränderung kann so vom Kunden beurteilt werden. Wenn nötig muss die Anwendungsmischung anschließend wieder auf die entsprechenden Stellen auf der Faser aufgetragen werden, um den Aufhellprozess fortzusetzen. Der Vorgang muss gegebenenfalls für weitere Kontrollen wiederholt werden. Die Anwendung transparenter Anwendungsmischungen vereinfacht diesen Kontrollschritt erheblich. Ein Abtragen des Blondiermittels von der Faser ist nicht notwendig. Stattdessen ermöglicht die Transparenz der Anwendungsmischung eine direkte, visuelle Beurteilung des Entfärbevorgangs zu jedem beliebigen Zeitpunkt während der Einwirkzeit. Die Bereitstellung transparenter Blondiermittel führt folglich zu einer verbesserten Handhabung des Blondiermittels und zu einer Vereinfachung der Anwendung.

Die DE 10 2007 041 490 A1 offenbart Blondiermittel, die die Beobachtung des Forschritts des Aufhellungsprozesses ohne arbeitsintensive Schritte und ohne die Gefahr der Beeinträchtigung des Blondierergebnisses ermöglichen.

WO 2005/067874 A1 beschreibt Blondiermittel, welche eine Mischung aus einem Oxidationsmittel, mindestens einem Stabilisator, mindestens einem Polymerverdicker aus synthetischen Polymeren und Alkalimagnesiumsilikaten und Wasser oder ein wässriges Lösungsmittel enthalten. Als wünschenswerte Eigenschaften des Mittels gemäß dieser Erfindung sind "Transparenz" und "angedickte Konsistenz" angegeben.

Die DE 10 2010 042 252 A1 offenbart Mittel zum Aufhellen keratinischer Fasern, welche mindestens zwei getrennt voneinander verpackte Zubereitungen (A) und (B) sowie gegebenenfalls eine weitere getrennt von (A) und (B) verpackte Zubereitung (C) enthalten, die unmittelbar vor der Anwendung zu einer Anwendungsmischung vermischt werden, wobei die Zubereitungen (A) mindestens ein Persulfat enthalten und die Zubereitungen (B) fließfähig sind und mindestens ein Oxidationsmittel enthalten und wobei Zubereitungen (B) und/oder Zubereitungen (C) weiterhin mindestens ein natürliches Polymer enthalten.

Die DE 10 2010 030337 A1 offenbart Mittel zum Aufhellen von keratinischen Fasern in Form einer getrennt voneinander verpackten Mehrkomponentenverpackungseinheit, umfassend eine Zubereitung (A), enthaltend ein Persulfat, eine Zubereitung (B), enthaltend ein Oxidationsmittel sowie ggf. eine Zubereitung (C).

In der DE 10 2004 061 468 A1 werden Abgabesysteme für haarfarbverändernde Mittel offenbart, welche mindestens zwei Behälter umfassen, enthaltend Wasserstoffperoxid, Silikonöl, kationisierte Proteinhydrolysate usw..

Nach der Lehre der US 2006/210499 A1 enthalten milde Bleichmittelzusammensetzungen Persulfate, ein Oxidationsmittel, sowie optional Keratinhydrolysat und Silikonöl.

Die DE 103 15 421 A1 offenbart Mittel zur Behandlung keratinhaltiger Fasern, enthaltend Wasserstoffperoxid und Persulfate sowie ferner Silikonöle und Keratinhydrolysate mit Molmassen im Bereich von 400 bis 800 Dalton.

In der DE 10 2006 008 149 A1 werden Bleichmittel für Haare offenbart, welche ein Oxidationsmittel, Persulfat und Keratinhydrolysate mit Molmassen im Bereich von 400 bis 800 Dalton enthalten.

Die US 2011/126361 A1 beschreibt Mittel zum Blondieren menschlicher Haare , enthaltend mindestens zwei getrennt voneinander verpackte Zubereitungen, welche Persulfate, Wasserstoffperoxid, Cellulose Gum sowie Silikon enthalten.

Die oxidative Behandlung keratinischer Fasern führt nicht nur zu dem gewünschten Aufhellergebnis, sie belastet die Fasern und kann die Faserstruktur im schlimmsten Falle schädigen. Um diese negativen Auswirkungen zu minimieren, werden in herkömmlichen nicht erfindungsgemäßen nicht transparenten Blondiermitteln Pflegestoffe eingesetzt, die reparierende und konditionierende Wirkungen besitzen. Viele der üblicherweise eingesetzten Pflegestoffe führen in transparenten Blondiermitteln aber zu einem Verlust oder zu einer deutlichen Beeinträchtigung der Transparenz, was den Anwendungsvorteil dieser Mittel konterkariert. Andere Pflegestoffe schwächen die Blondierleistung, weshalb deren Einsatz kontraindiziert ist.

Aufgabe der vorliegenden Erfindung war es, die Eigenschaften transparenter Blondiermittel in Bezug auf ihre pflegenden Eigenschaften zu verbessern, ohne ihre Leistung oder ihre Transparenz zu beeinträchtigen.

Es hat sich gezeigt, dass mit einem natürlichen Polymer verdickte Oxidationsmittelzubereitungen sich mit Blondierpulvern zu einer fließfähigen Zubereitung vermischen lassen, welche eine gute Homogenisierung der beiden Komponenten zu einer fertig vermischten Anwendungsmischung gestattet. In dieser speziellen Matrix führen bestimmte Proteine in Kombination mit/ohne Silikonöl zu permanenten Pflegeeffekten, ohne die Transparenz zu beeinträchtigen. Darüber hinaus wird die Blondierleistung verstärkt.

Gegenstand der vorliegenden Erfindung sind in einer ersten Ausführungsform Mittel zum Aufhellen keratinischer Fasern, enthaltend mindestens zwei getrennt voneinander verpackte Zubereitungen (A) und (B) sowie gegebenenfalls eine weitere getrennt von (A) und (B) verpackte Zubereitung (C), die unmittelbar vor der Anwendung zu einer Anwendungsmischung vermischt werden, wobei
i. Zubereitung (A) mindestens ein Persulfat enthält und
ii. Zubereitung (B) fließfähig ist und mindestens ein Oxidationsmittel enthält,
iii. Zubereitung (B) und/oder Zubereitung (C) weiterhin mindestens ein natürliches Polymer enthält/enthalten,
und Zubereitung (A) - bezogen auf ihr Gewicht -
a1) 0 bis 3 Gew.-% Keratinhydrolysat(e) und/oder
a2) 0 bis 5 Gew.-% Silikonöl(e) enthält,
mit der Maßgabe, dass die Zubereitung (A) - bezogen auf ihr Gewicht - 0,1 bis 6 Gew.-% Verbindung(en) aus den Gruppen a1) und a2) enthält,
wobei die Zubereitung (A) 0,01 bis 1 Gew.-% Hydrolysat(e) mit Molmassen von 400 bis 1200 Dalton, gewonnen aus dem Kortex und/oder der Cuticula von keratinischen Fasern, enthält.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Aufhellen von Keratinfasern geeignet sind, steht prinzipiell einer Verwendung auch auf anderen Gebieten nichts entgegen.

Die Zubereitungen (A) sind bevorzugt pulverförmig. Dabei können Pulver aus festen Bestandteilen mit unterschiedlichen Korngrößen eingesetzt werden. Üblicherweise kann es bevorzugt sein, wenn die Pulver jedoch eine möglichst homogene Korngröße aufweisen, insbesondere um eine einheitliche Dispersion bzw. Auflösung der Pulver in den Zubereitungen (B) zu erleichtern.

Die Zubereitungen (A) können die Wirkstoffe in einem festen kosmetischen Träger enthalten. Ein fester kosmetischer Träger kann Salze der Kieselsäure, insbesondere Salze der Silicate und Metasilicate mit Ammonium, Alkalimetallen sowie Erdalkalimetallen enthalten. Insbesondere Metasilicate, die sich gemäß Formel (SiO₂)ₙ(M₂O)ₘ, wobei M für ein Ammoniumion, ein Alkalimetall oder ein halbes Stöchiometrieäquivalents eines Erdalkalimetalls steht, durch das Verhältnis zwischen n und m von ≤ 1 auszeichnen und sich als kettenförmige polymere Strukturen des Anions [SiO₃]²⁻ auffassen lassen, können bevorzugt eingesetzt werden. Natriummetasilicat der Formel [Na₂SiO₃]∞, ist dabei besonders bevorzugt. Erfindungsgemäß gleichfalls bevorzugt sind solche Silicate, die aus einem Silicat der Formel (SiO₂)ₙ(Na₂O)ₘ(K₂O)ₚ gebildet werden, wobei n für eine positive rationale Zahl und m und p unabhängig voneinander für eine positive rationale Zahl oder für 0 stehen, mit den Maßgaben, dass mindestens einer der Parameter m oder p von 0 verschieden ist und das Verhältnis zwischen n und der Summe aus m und p zwischen 2:1 und 4:1 liegt.

Weiterhin können die festen kosmetischen Träger so genannte Rieselhilfen enthalten, die ein Verklumpen oder Verbacken der Pulver-Bestandteile verhindern sollen. Als solche Rieselhilfen kommen bevorzugt wasserunlösliche, hydrophobierende oder Feuchtigkeit adsorbierende Pulver von Kieselgur, pyrogenen Kieselsäuren, Calciumphosphat, Calciumsilicaten, Aluminiumoxid, Magnesiumoxid, Magnesiumcarbonat, Zinkoxid, Stearaten, Fettaminen und dergleichen in Frage. Schließlich können die festen kosmetischen Träger noch zusätzlich ein Entstaubungsmittel enthalten, die die Staubbildung der pulverförmigen Bestandteile verhindert. Hierzu können insbesondere inerte Öle eingesetzt werden. Bevorzugtenthalten die festen, kosmetischen Träger als Entstaubungsmittel Esteröle oder Mineralöle, bevorzugt Kohlenwasseröle, wie flüssiges Paraffinöl.

Als ersten wesentlichen Inhaltsstoff enthält die Zubereitung (A) mindestens ein Persulfat. Erfindungsgemäß geeignete Persulfate sind anorganische Peroxoverbindungen. Diese sind bevorzugt ausgewählt aus Ammoniumperoxodisulfat, Alkalimetallperoxodisulfaten, Ammoniumperoxomonosulfat, Alkalimetallperoxomonosulfaten, Alkalimetallperoxodiphosphaten und/oder Erdalkalimetallperoxiden. Besonders bevorzugt sind Ammoniumperoxodisulfat und/oder Alkalimetallperoxodisulfate.

In einer bevorzugten Ausführungsform der vorliegenden Erfindung enthält Zubereitung (A) als Persulfat mindestens ein Peroxodisulfat-Salz, insbesondere ausgewählt aus Ammoniumperoxodisulfat und/oder Kaliumperoxodisulfat und/oder Natriumperoxodisulfat. Weiterhin hat es sich bei den Arbeiten zur vorliegenden Erfindung als besonders bevorzugt erwiesen, wenn die Zubereitungen (A) mindestens zwei verschiedene Peroxodisulfate enthalten. Bevorzugte Peroxodisulfat-Salze sind dabei Kombinationen von Ammoniumperoxodisulfat mit Kaliumperoxodisulfat und/oder Natriumperoxodisulfat.

Die Zubereitungen (A) enthalten vorzugsweise Persulfat-Salze in einer Menge von 0,1 bis 80 Gew.-%, bevorzugt von 2 bis 50 Gew.-%, besonders bevorzugt von 3 bis 30 Gew.-% und insbesondere bevorzugt 5 bis 15 Gew.-% explizit 5, 6, 7, 8, 9, 10, 11, 12, 13, 14 oder 15 Gew.-% jeweils bezogen auf das Gesamtgewicht des Mittels.

Erfindungsgemäß enthalten die Zubereitung (B) und/oder Zubereitung (C) weiterhin mindestens ein natürliches Polymer (siehe unten). Zubereitung (A) ist vorzugsweise frei von Xanthan, da es sich gezeigt hat, dass dies die Aufhellungsleistung verbessert.

Erfindungsgemäß bevorzugte Mittel sind dadurch gekennzeichnet, dass Zubereitung (A) frei von Xanthan ist und - bezogen auf ihr Gewicht - 5 bis 60 Gew.-%, vorzugsweise 10 bis 55 Gew.-%, besonders bevorzugt 15 bis 50 Gew.-% und insbesondere 20 bis 45 Gew.-% mindestens eines Persulfat, ausgewählt aus Ammoniumperoxodisulfat und/oder Kaliumperoxodisulfat und/oder Natriumperoxodisulfat, enthält.

Erfindungsgemäß enthält Zubereitung (A) - bezogen auf ihr Gewicht-
a1) 0 bis 3 Gew.-% Keratinhydrolysat(e) und/oder
a2) 0 bis 5 Gew.-% Silikonöl(e),
mit der Maßgabe, dass die Zubereitung (A) - bezogen auf ihr Gewicht - 0,1 bis 6 Gew.-% Verbindung(en) aus den Gruppen a1) und a2) enthält,
wobei die Zubereitung (A) 0,01 bis 1 Gew.-% Hydrolysat(e) mit Molmassen von 400 bis 1200 Dalton, gewonnen aus dem Kortex und/oder der Cuticula von keratinischen Fasern, enthält.

Erfindungsgemäße Mittel können demnach in der Zubereitung (A) ausschließlich Keratinhydrolysat(e) enthalten - dann in Mengen von 0,1 bis 3 Gew.-%, oder sowohl Keratinhydrolysat(e) als auch Silikonöl(e) - dann in Gesamtmengen beider Inhaltsstoffe von 0,1 bis 6 Gew.-%, jeweils bezogen auf das Gewicht der Zubereitung (A).

Erfindungsgemäße Mittel sind dadurch gekennzeichnet, dass die Zubereitung (A) 0,01 bis 1 Gew.-%, vorzugsweise 0,05 bis 0,75 Gew.-%, weiter bevorzugt 0,1 bis 0,5 Gew.-%, besonders bevorzugt 0,15 bis 0,4 Gew.-% und insbesondere 0,2 bis 0,3 Gew.-% Hydrolysat(e) mit Molmassen von 400 bis 1200 Dalton, gewonnen aus dem Kortex und/oder der Cuticula von keratinischen Fasern, enthält.

Weitere erfindungsgemäß bevorzugte Mittel sind dadurch gekennzeichnet, dass die Zubereitung (A) 0,01 bis 5 Gew.-%, vorzugsweise 0,05 bis 4 Gew.-%, weiter bevorzugt 0,1 bis 2,5 Gew.-%, besonders bevorzugt 0,25 bis 2 Gew.-% und insbesondere 0,5 bis 1,5 Gew.-% Silikonöl(e) aus der Gruppe der Verbindungen mit der INCI-Bezeichnung Dimethicone enthält.

Dimethicone können sowohl linear als auch verzweigt als auch cyclisch oder cyclisch und verzweigt sein. Lineare Dimethicone können durch die folgende Strukturformel (Si1) dargestellt werden:

(SiR¹₃)-O-(SiR²₂-O-)ₓ-(SiR¹₃) (Si1)

Verzweigte Dimethicone können durch die Strukturformel (Si1.1) dargestellt werden:

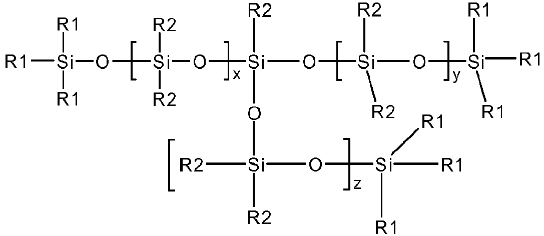

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH₂-, -CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H₄C₆H₄-, -C₆H₄CH₂C₆H₄-; und -(CH ₂)₃C(O)SCH₂CH₂- ein. Bevorzugt als R¹ und R² sind Methyl, Phenyl und C2 bis C22 - Alkylreste. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs. Höchst bevorzugt sind Viskositäten um den Bereich von etwa 60.000 cPs herum. Das Wort "etwa" definiert dabei eine dem Fachmann bei technisch hergestellten Produkten übliche Abweichung von dem genannten Wert im Anschluß an das Wort "etwa". Beispielhaft sei hier auf das Produkt "Dow Corning 200 mit 60000cSt" verwiesen. Selbstverständlich umfasst die erfindungsgemäße Lehre auch, dass die Dimethicone bereits als Emulsion vorliegen können.

Wenn die Dimethicone als Emulsion verwendet werden, dann beträgt die Tröpfchengröße der emulgierten Teilchen erfindungsgemäß 0,01µm bis 10000 µm, bevorzugt 0,01 bis 100 µm, ganz besonders bevorzugt 0,01 bis 20 µm und am bevorzugtesten 0,01 bis 10 µm. Die Teilchengröße wird dabei nach der Methode der Lichtstreuung bestimmt.

Besonders bevorzugte erfindungsgemäße Mittel sind dadurch gekennzeichnet, dass die Zubereitung (A) 0,01 bis 5 Gew.-%, vorzugsweise 0,05 bis 4 Gew.-%, weiter bevorzugt 0,1 bis 2,5 Gew.-%, besonders bevorzugt 0,25 bis 2 Gew.-% und insbesondere 0,5 bis 1,5 Gew.-% mindestens eines Silikons der Formel (Si-I)

(CH₃)₃Si-[O-Si(CH₃)₂]ₓ-O-Si(CH₃)₃ (Si-I),

enthalten, in der x für eine Zahl von 0 bis 100, vorzugsweise von 0 bis 50, weiter bevorzugt von 0 bis 20 und insbesondere 0 bis 10, steht.

Weitere erfindungsgemäß bevorzugte Mittel sind dadurch gekennzeichnet, dass die Zubereitung (A) 0,01 bis 5 Gew.-%, vorzugsweise 0,05 bis 4 Gew.-%, weiter bevorzugt 0,1 bis 2,5 Gew.-%, besonders bevorzugt 0,25 bis 2 Gew.-% und insbesondere 0,5 bis 1,5 Gew.-% Silikonöl(e) aus der Gruppe der Verbindungen mit der INCI-Bezeichnung Amodimethicone enthält, wobei Verbindungen mit den INCI-Bezeichnungen Silicone Quaternium-1, Silicone Quaternium-2, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, Silicone Quaternium-16, Silicone Quaternium-17, Silicone Quaternium-18, Silicone Quaternium-20, Silicone Quaternium-21 oder Silicone Quaternium-22 bevorzugt sind.

Es hat sich gezeigt, dass insbesondere 4-Morpholinomethyl-substituierte Silikone zu besonders guten Pflegeergebnissen führen, ohne die Transparenz der Mittel zu beeinträchtigen. Besonders bevorzugte erfindungsgemäße Mittel sind dadurch gekennzeichnet, dass die Zubereitung (A) 0,01 bis 5 Gew.-%, vorzugsweise 0,05 bis 4 Gew.-%, weiter bevorzugt 0,1 bis 2,5 Gew.-%, besonders bevorzugt 0,25 bis 2 Gew.-% und insbesondere 0,5 bis 1,5 Gew.-% Silikonöl(e) der Formel (I) enthält, 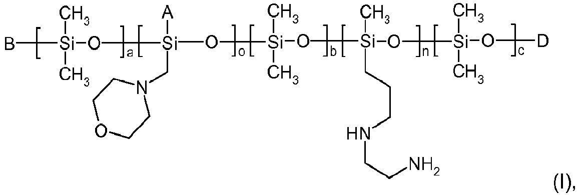 in der
A für eine über ein -O- gebundene Struktureinheit (I), (II) oder (III) 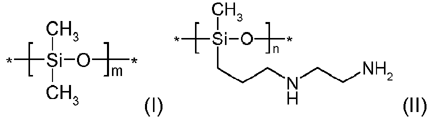 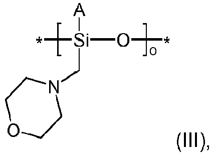
oder einen über ein -O- gebundenen oligomeren oder polymeren Rest enthaltend Struktureinheiten der Formeln (I), (II) oder (III) oder die Hälfte eines verbindenden O-Atoms zu einer Struktureinheit (III) oder für -OH steht,
* für eine Bindung zu einer der Struktureinheiten (I), (II) oder (III) oder für eine Endgruppe B (Si-gebunden) oder D (O-gebunden) steht,
B für eine Gruppe -OH, -O-Si(CH₃)₃,-O-Si(CH₃)₂OH, -O-Si(CH₃)₂OCH₃ steht,
D für eine Gruppe -H, -Si(CH₃)₃,-Si(CH₃)₂OH, -Si(CH₃)₂OCH₃ steht,
a, b und c für ganze Zahlen zwischen 0 und 1000 stehen, mit der Maßgabe a + b + c > 0
m, n und o für ganze Zahlen zwischen 1 und 1000 stehen.

Solche bevorzugten Mittel enthalten in der Zubereitung (A) 0,01 bis 5 Gew.-%, vorzugsweise 0,05 bis 4 Gew.-%, weiter bevorzugt 0,1 bis 2,5 Gew.-%, besonders bevorzugt 0,25 bis 2 Gew.-% und insbesondere 0,5 bis 1,5 Gew.-% mindestens eines 4-morpholinomethyl-substituierten Silikons der Strukturformel (I). Diese verdeutlicht, dass die Siloxangruppen n und o nicht zwingend direkt an eine Endgruppierung B bzw. D gebunden sein müssen. Vielmehr gilt in bevorzugten Formeln (I) a > 0 oder b > 0 und in besonders bevorzugten Formeln (I) a > 0 und b > 0, d.h. die terminale Gruppierung B bzw. D ist vorzugsweise an eine Dimethylsiloxy-Gruppierung gebunden. Auch in Formel (I) sind die Siloxaneinheiten a, b, c, n und o vorzugsweise statistisch verteilt.

Die durch Formel (I) dargestellten erfindungsgemäß eingesetzten Silikone können beidseitig trimethylsilyl-terminiert sein (D = -Si(CH₃)₃, B = -O-Si(CH₃)₃), sie können aber auch ein- oder zweiseitig dimethylsilylhydroxy- oder dimethylsilylmethoxy-terminiert sein. Im Rahmen der vorliegenden Erfindung besonders bevorzugt eingesetzte Silikone weisen mindestens eine endständige Dimethylsilylhydroxy-Gruppe auf, d.h. sind ausgewählt aus Silikonen, in denen
B = -O-Si(CH₃)₂OH und D = -Si(CH₃)₃
B = -O-Si(CH₃)₂OH und D = -Si(CH₃)₂OH
B = -O-Si(CH₃)₂OH und D = -Si(CH₃)₂OCH₃
B = -O-Si(CH₃)₃ und D = -Si(CH₃)₂OH
B = -O-Si(CH₃)₂OCH₃ und D = -Si(CH₃)₂OH bedeutet. Diese Silikone führen zu exorbitanten Verbesserungen der Haareigenschaften der mit den erfindungsgemäßen Mitteln behandelten Haare, insbesondere zu einer gravierenden Verringerung des Kontaktwinkels.

Die Struktureinheiten der Formeln (I), (II) und (III) können statistisch verteilt im Molekül vorliegen, die erfindungsgemäß eingesetzten Silikone können aber auch Blockcopolymere aus Blöcken der einzelnen Struktureinheiten sein, wobei die Blöcke wiederum statistisch verteilt vorliegen können. Der * an den freien Valenzen der Struktureinheiten (I), (II) oder (III) steht dabei für eine Bindung zu einer der Struktureinheiten (I), (II) oder (III) oder für eine Endgruppe B (Si-gebunden) oder D (O-gebunden).

In Formel (I) kann der Rest A
- für eine über ein -O- gebundene Struktureinheit (I), (II) oder (III) oder
- einen über ein -O- gebundenen oligomeren oder polymeren Rest enthaltend Struktureinheiten der Formeln (I), (II) oder (III)
- oder die Hälfte eines verbindenden O-Atoms zu einer Struktureinheit (III) oder für -OH stehen.

Damit wird Formel (I) präzisiert zu einer der Formeln (la), (Ib), (lc), (Id), (Ie) oder (If):

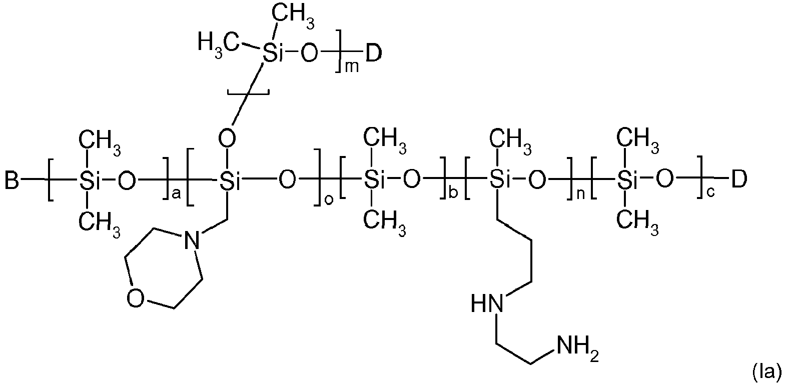

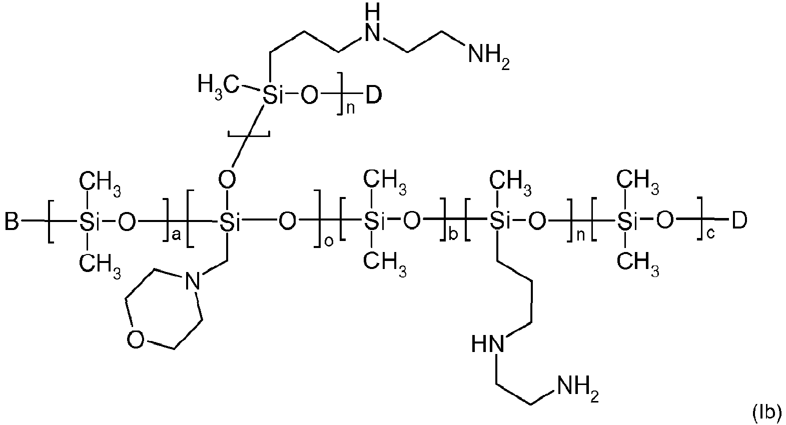

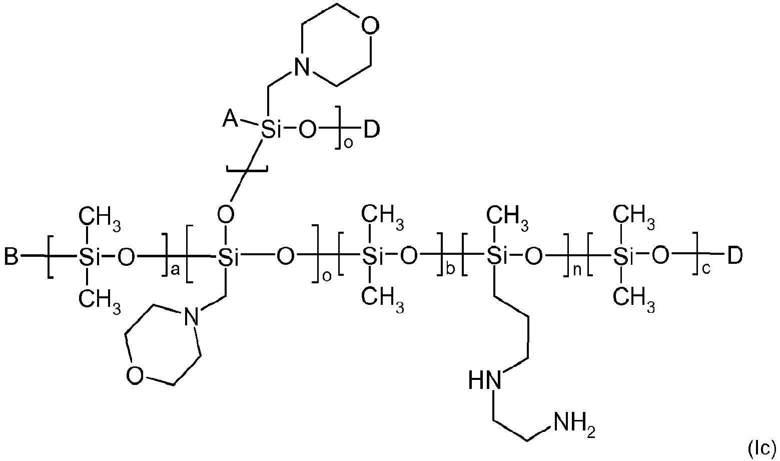

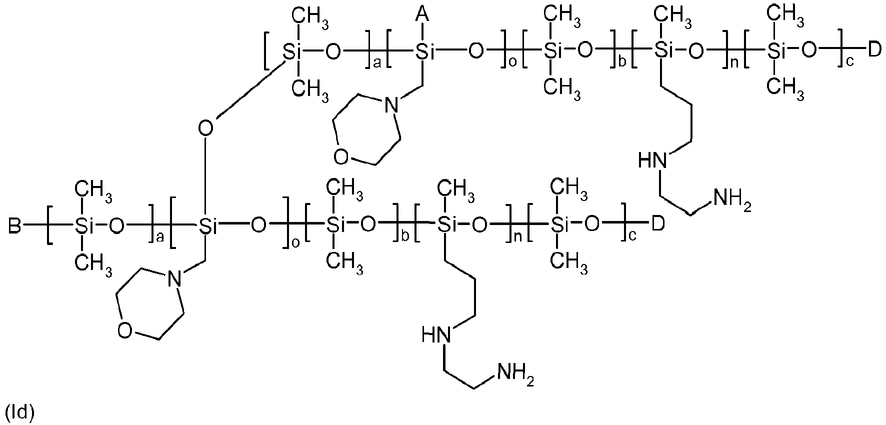

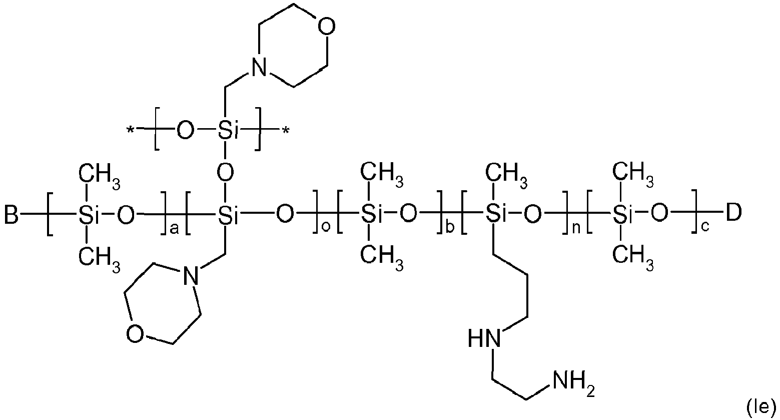

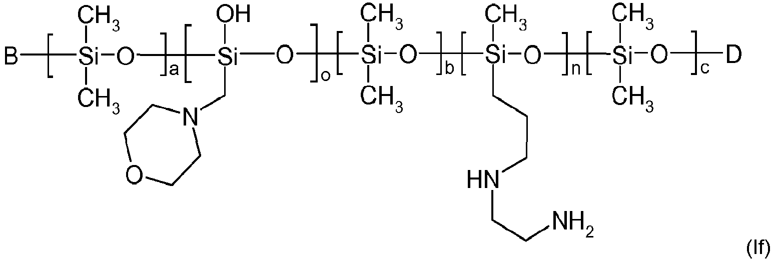

In der Struktureinheit (III) kann der Rest A
- für eine über ein -O- gebundene Struktureinheit (I), (II) oder (III) oder
- einen über ein -O- gebundenen oligomeren oder polymeren Rest enthaltend Struktureinheiten der Formeln (I), (II) oder (III)
- oder die Hälfte eines verbindenden O-Atoms zu einer Struktureinheit (III) oder für -OH stehen.

Im erstgenannten Fall wird die Struktureinheit (III) zu einer der Struktureinheiten (IIIa), (IIIb) oder (IIIc): 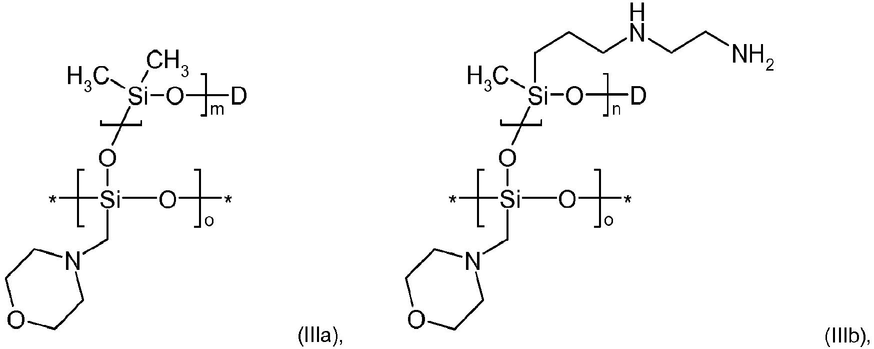 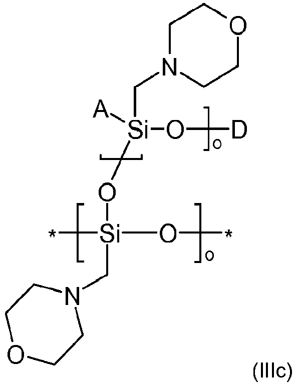 mit m = n = o = 1 und A bzw. D wie vorstehend definiert.

Im zweiten Fall können in den vorstehend genannten Formeln (IIIa), (IIIb) und (IIIc) die Indices m, n und o für ganze Zahlen zwischen 2 und 1000 stehen. Der zweite Fall deckt aber auch oligomere oder polymere Reste, ab, die mindestens zwei verschiedene Struktureinheiten der Formeln (I), (II) oder (III) enthalten, wie in Formel (Illd) dargestellt: 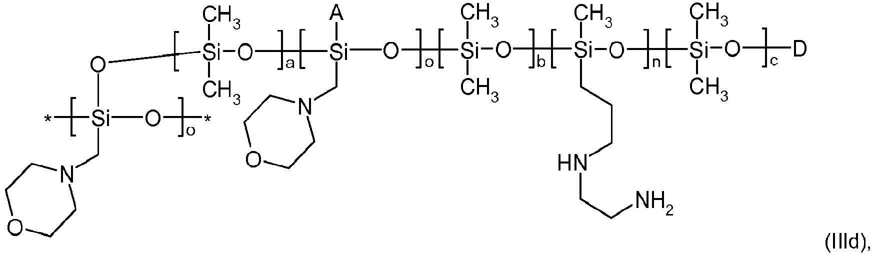 in der a, b und cfür ganze Zahlen zwischen 0 und 1000 stehen, mit der Maßgabe a + b + c > 0 sowie n und o für ganze Zahlen zwischen 1 und 1000 stehen.

Im dritten Fall steht A die Hälfte eines verbindenden O-Atoms zu einer Struktureinheit (III) (dargestellt in der Struktureinheit (IIIe) oder für -OH (dargestellt in der Struktureinheit (IIIf)

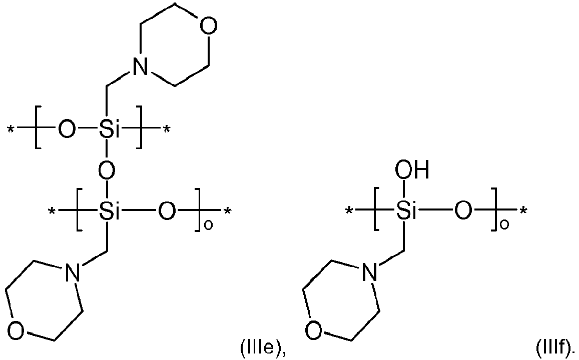

Die Struktureinheit (III) bzw. die Siloxaneinheiten o in der Formel (I) können über die Gruppe A Nest- bzw. Teilkäfigstrukturen ausbilden, wenn A für die Hälfte eines verbindenden O-Atoms zu einer Struktureinheit (III) steht. Erfindungsgemäße Haarbehandlungsmittel, die Silikone mit entsprechenden 4-morpholinomethyl-substituierten Silsequioxan-Teilstrukturen beinhalten, sind erfindungsgemäß bevorzugt, da diese Silikone zu enorm verbesserten Kämmbarkeiten und drastisch reduzierten Kontaktwinkeln führen.

Erfindungsgemäß bevorzugte Mittel sind demnach dadurch gekennzeichnet, dass die Zusammensetzung (A) mindestens ein 4-morpholinomethyl-substituiertes Silikon enthält, welches Struktureinheiten der Formel (II) aufweist 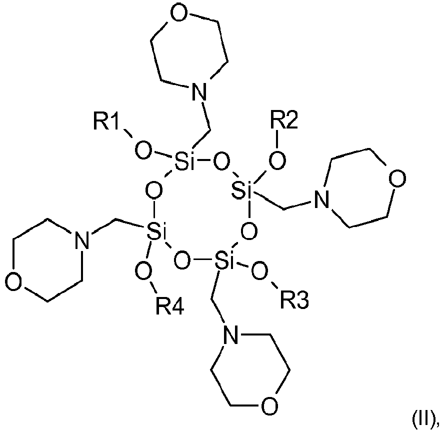 in der
R1, R2, R3 und R4 unabhängig voneinander für -H, -CH₃, eine Gruppe D, eine Struktureinheit (I), (II) oder (III) oder einen oligomeren oder polymeren Rest enthaltend Struktureinheiten der Formeln (I), (II) oder (III) stehen oder zwei der Reste R1, R2, R3 und R4 für eine Struktureinheit -Si(R6)(R5)- mit
- R5 =: -CH₃ oder eine Struktureinheit der Formel (I) oder (II) oder (III) oder einen oligomeren oder polymeren Rest enthaltend Struktureinheiten der Formeln (I), (II) oder (III)
- R6 =: -OH, -CH₃, oder eine Struktureinheit der Formel (I) oder (II) oder (III) oder einen oligomeren oder polymeren Rest enthaltend Struktureinheiten der Formeln (I), (II) oder (III).

In bevorzugten Silikonen der Formel (II) steht mindestens einer der Reste R1, R2, R3 oder R4 für einen oligomeren oder polymeren Rest enthaltend Struktureinheiten der Formeln (I), (II) oder (III).

In weiter bevorzugten Silikonen der Formel (II) steht mindestens einer der Reste R1, R2, R3 oder R4 für einen oligomeren oder polymeren Rest enthaltend Struktureinheiten der Formeln (I) und (II). In noch weiter bevorzugten Silikonen der Formel (II) steht mindestens einer der Reste R1, R2, R3 oder R4 für einen oligomeren oder polymeren Rest enthaltend Struktureinheiten der Formeln (I) und (II) und (III).

Vorzugsweise steht mindestens einer der Reste R1, R2, R3 oder R4 für eine -[-Si(CH₃)₂-O]ₘ-Gruppierung, d.h. ein Oligo- bzw. Polymeres der Struktureinheit (I). Vorzugsweise ist darüber hinaus die Struktureinheit (II) bzw. ein Oligo- oder Polymeres davon nie allein, sondern immer in statistischer Verteilung mit weiteren Struktureinheiten der Formel (I) als einer der Reste R1, R2, R3 oder R4 im Molekül gebunden.

Unabhängig davon, welches spezielle 4-Morpholinomethyl-substituierte Silikon in der Zubereitung (A) eingesetzt wird, sind erfindungsgemäße Mittel bevorzugt, die in der Zubereitung (A) ein 4-Morpholinomethyl-substituiertes Silikon enthalten, in dem mehr als 50 Mol-% der Struktureinheiten Dimethylsiloxy-Einheiten sind, d.h. in denen die Struktureinheit (I) mindestens die Hälfte aller Struktureinheiten des eingesetzten Silikons ausmacht.

In anderen Worten sind Silikone bevorzugt, bei denen m > (n + o) bzw. (a+b+c) > (n + o) gilt.

Noch weiter bevorzugte Mittel enthalten in der Zubereitung (A) ein 4-Morpholinomethyl-substituiertes Silikon, in dem mehr als 90 Mol-% der Struktureinheiten Dimethylsiloxy-Einheiten sind, d.h. in denen die Struktureinheit (I) mindestens Neun Zehntel aller Struktureinheiten des eingesetzten Silikons ausmacht.

In anderen Worten sind Silikone bevorzugt, bei denen m > 10(n + o) bzw. (a+b+c) > 10(n + o) gilt.

Noch weiter bevorzugte Kosmetische Mittel enthalten ein 4-Morpholinomethyl-substituiertes Silikon, in dem mehr als 98 Mol-% der Struktureinheiten Dimethylsiloxy-Einheiten sind, d.h. in denen die Struktureinheit (I) mindestens Achtundneunzig Hundertstel aller Struktureinheiten des eingesetzten Silikons ausmacht.

In anderen Worten sind Silikone bevorzugt, bei denen m > 50(n + o) bzw. (a+b+c) > 50(n + o) gilt. Noch weiter bevorzugte Kosmetische Mittel enthalten ein 4-Morpholinomethyl-substituiertes Silikon, in dem mehr als 98,5 Mol-% der Struktureinheiten Dimethylsiloxy-Einheiten sind, d.h. in denen die Struktureinheit (I) mindestens Neunhundertfünfundachtzig Tausendstel aller Struktureinheiten des eingesetzten Silikons ausmacht.

In anderen Worten sind Silikone bevorzugt, bei denen m > 75(n + o) bzw. (a+b+c) > 75(n + o) gilt.

Noch weiter bevorzugte Kosmetische Mittel enthalten ein 4-Morpholinomethyl-substituiertes Silikon, in dem mehr als 99 Mol-% der Struktureinheiten Dimethylsiloxy-Einheiten sind, d.h. in denen die Struktureinheit (I) mindestens Neun Zehntel aller Struktureinheiten des eingesetzten Silikons ausmacht.

In anderen Worten sind Silikone bevorzugt, bei denen m > 100(n + o) bzw. (a+b+c) > 100(n + o) gilt.

Zusammenfassend sind bevorzugte erfindungsgemäße Mittel dadurch gekennzeichnet, dass sie in der Zubereitung (A) mindestens ein 4-morpholinomethyl-substituiertes Silikon enthalten, bei dem
- m > (n + o) bzw. (a+b+c) > (n + o), vorzugsweise
- m > 10(n + o) bzw. (a+b+c) > 10(n + o), besonders bevorzugt
- m > 50(n + o) bzw. (a+b+c) > 50(n + o), weiter bevorzugt
- m > 75(n + o) bzw. (a+b+c) > 75(n + o) und insbesondere
- m > 100(n + o) bzw. (a+b+c) > 100(n + o) gilt.

Es hat sich gezeigt, dass die Wirkung der erfindungsgemäß eingesetzten Silikone noch gesteigert werden kann, wenn bestimmte nichtionische Komponenten ebenfalls in den erfindungsgemäßen Mitteln eingesetzt werden. Zudem haben diese nichtionischen Komponenten positive Effekte auf die Lagerstabilität der erfindungsgemäßen Mittel. Nichtionische Komponenten, die hier besonders geeignet sind, sind Ethoxylate von Decanol, Undecanol, Dodecanol, Tridecanol usw.. Als besonders geeignet haben sich ethoxylierte Tridecanole erwiesen, die mit besonderem Vorzug in die erfindungsgemäßen Mittel inkorporiert werden. Erfindungsgemäß besonders bevorzugte kosmetische Zusammensetzungen enthalten - bezogen auf ihr Gewicht - 0,00001 bis 5 Gew.-%, vorzugsweise 0,0001 bis 3,5 Gew.-%, besonders bevorzugt 0,001 bis 2 Gew.-%, weiter bevorzugt 0,01 bis 1 Gew.-% und insbesondere 0,1 bis 0,5 Gew.-% verzweigtes, ethoxyliertes Tridecanol (INCI-Bezeichnung: Trideceth-5) oder α-iso-Tridecyl-ω-hydroxypolyglycolether (INCI-Bezeichnung: Trideceth-10) oder deren Mischungen.

Erfindungsgemäß bevorzugte morpholinomethyl-substituierte(s) Silikon(e) weisen sowohl Hydroxyals auch Alkoxygruppen auf. Erfindungsgemäß besonders bevorzugte Mittel enthalten in der Zubereitung (A) hydroxy-terminierte(s) 4-morpholinomethyl-substituierte(s) Silikon(e), in denen das Molverhältnis Hydroxy/Alkoxy im Bereich von 0,2:1 bis 0,4:1, vorzugsweise im Bereich von 1:0,8 bis 1:1,1 liegt.

Die mittleren Molekulargewicht des Silikons beträgt vorzugsweise von 2.000 bis 200.000 und noch mehr bevorzugt von 5.000 bis 100.000, insbesondere 10.000 bis 50.000 Dalton. Kosmetische Zusammensetzungen, bei denen die gewichtmittlere Molmasse des in ihnen enthaltenen 4-morpholinomethyl-substituierten Silikons der Formel (I) im Bereich von 2.000 bis 1.000.000 gmol⁻¹, vorzugsweise im Bereich von 5.000 bis 200.000 gmol⁻¹ liegt, sind bevorzugt.

Die mittleren Molekulargewichte von aminosubstituierten Silikonen sind beispielsweise durch Gelpermeationschromatographie (GPC) bei Raumtemperatur in Polystyrol messbar. Als Säulen können Styragel Spalten µ, als Eluent THF und als Flussrate 1 ml / min gewählt werden. Die Detektion erfolgt vorzugsweise mittels Refraktometrie und UV-Meter.

Der erste Gegenstand der Erfindung umfasst Mittel zum Aufhellen keratinischer Fasern, die mindestens zwei getrennt voneinander verpackte Zubereitungen (A) und (B) sowie gegebenenfalls eine weitere getrennt von (A) und (B) verpackte Zubereitung (C) enthalten. Die Zubereitungen (B) und gegebenenfalls (C) enthalten die Wirkstoffe in einem fließfähigen, kosmetischen Träger. Die Grundlage des fließfähigen, kosmetischen Trägers ist dabei bevorzugt wässrig oder wässrigalkoholisch. Zum Zwecke der Haarbleiche sind solche Träger beispielsweise transparente Gele oder auch tensidhaltige schäumende Lösungen, wie beispielsweise Shampoos, Schaumaerosole oder andere Zubereitungen, die für die Anwendung auf dem Haar geeignet sind. Ein bevorzugter, fließfähiger Träger enthält im Sinne der Erfindung mindestens 40 Gew.-%, insbesondere mindestens 50 Gew.-% Wasser. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether enthalten. Bevorzugt sind dabei alle wasserlöslichen organischen Lösungsmittel.

Als wesentlichen Inhaltstoff enthalten die Zubereitungen (B) und/oder die Zubereitungen (C) des Blondiermittels gemäß der Erfindung mindestens ein natürliches Polymer.

Wenn das Mittel zum Aufhellen keratinischer Fasern, genau zwei getrennt voneinander verpackte Zubereitungen (A) und (B) enthält, die unmittelbar vor der Anwendung zu einer Anwendungsmischung vermischt werden, so enthält Zubereitung (B) gemäß der Erfindung mindestens ein natürliches Polymer.

Wenn das Mittel zum Aufhellen keratinischer Fasern, mindestens drei getrennt voneinander verpackte Zubereitungen (A), (B) und (C) enthält, die unmittelbar vor der Anwendung zu einer Anwendungsmischung vermischt werden, so können Zubereitung (B) und/oder Zubereitung (C) mindestens ein natürliches Polymer enthalten.

Als natürliches Polymer können beispielsweise Cellulosederivate verwendet werden, die als Verdickungsmittel Verwendung finden. Beispiele sind Agar-Agar, Carrageen, Alginate, Xanthan-Gum, Karaya-Gummi, Ghatti-Gummi, Tragant, Skleroglucangums oder Gummi arabicum, Alginate, Pektine, Polyosen, Guar-Gums, Johannisbrotbaumkernmehl, Leinsamengummen, Dextrane, Pektine, Staerke-Fraktionen und Derivate wie Amylose, Amylopektin und Dextrine, Gelatine und Casein sowie Cellulosederivate, wie beispielsweise Methylcellulose, Carboxyalkylcellulosen wie Carboxymethylcellulose und Hydroxyalkylcellulosen wie Hydroxyethylcellulose.

Natürliche Polymere aus den genannten Substanzklassen sind kommerziell erhältlich und werden beispielsweise unter den Handelsnamen Deuteron^{®}-XG (anionisches Heteropolysaccharid auf Basis von β-D-Glucose, D-Manose, D-Glucuronsaeure, Schoener GmbH), Deuteron^{®}-XN (nichtionogenes Polysaccharid, Schoener GmbH), Protanal RF 6650 alginate (Natriumalginat, FMC Biopolymer), Cekol (Cellulose Gum, Kelco), Kelzan (Xanthan-Biopolymer, Kelco), Xanthan FN (Xanthan-Biopolymer, Jungbunzlauer), Keltrol z.B. Keltrol CG-T (Xanthan-Biopolymer, Kelco) oder Keltrol CG-SFT (Xanthan-Biopolymer, Kelco) angeboten.

In einer bevorzugten Ausführungsform der Erfindung enthalten die Zubereitungen (B) und/oder gegebenenfalls (C) Xanthan.

Erfindungsgemäß bevorzugt sind solche Xanthane, die nach der Quellung transparente Zubereitungen ergeben. Insbesondere bevorzugt ist die Verwendung des Xanthan-Biopolymers, welches unter dem Handelsnamen Keltrol CG-SFT der Firma Kelco vertrieben wird.

In einer bevorzugten Ausführungsform enthält Zubereitung (B) bezogen auf ihr Gewicht Xanthan in Mengen von 0,1 bis 10 Gew.-%, vorzugsweise von 0,5 bis 6 Gew.-%, besonders bevorzugt von 0,7 bis 5 Gew.-% und insbesondere bevorzugt von 1 bis 4 Gew.-%, explizit 1, 2, 3 oder 4 Gew.-%, wenn das Mittel zum Aufhellen keratinischer Fasern, genau zwei getrennt voneinander verpackte Zubereitungen (A) und (B) enthält, die unmittelbar vor der Anwendung zu einer Anwendungsmischung vermischt werden. Vorzugsweise enthalten die fertigen vermischten Anwendungszubereitungen bezogen auf ihr Gewicht, Xanthan in Mengen von 0,6 bis 5 Gew.-%, besonders bevorzugt von 1,0 bis 3,5 Gew.-% und insbesondere bevorzugt von 1,5 bis 2,5 Gew.-% explizit 1,5; 1,6; 1,7; 1,8; 1,9; 2,0; 2,1; 2,2; 2,3; 2,4 oder 2,5 Gew.-%.

Wenn das Mittel zum Aufhellen keratinischer Fasern, mindestens drei getrennt voneinander verpackte Zubereitungen (A), (B) und (C) enthält, die unmittelbar vor der Anwendung zu einer Anwendungsmischung vermischt werden, so können Zubereitung (B) und/oder Zubereitung (C) Xanthan enthalten. Wenn das Mittel zum Aufhellen keratinischer Fasern, mindestens drei getrennt voneinander verpackte Zubereitungen (A), (B) und (C) enthält, die unmittelbar vor der Anwendung zu einer Anwendungsmischung vermischt werden, sind Zubereitungen bevorzugt, in denen Zubereitung (C) Xanthan enthält. Insbesondere bevorzugt sind Zubereitungen, in denen Zubereitung (C) Xanthan enthält und Zubereitung (B) frei von Xanthan ist.

Unabhängig davon, ob Zubereitung (B) und/oder Zubereitung (C) Xanthan enthalten, sind solche fertig vermischten Anwendungszubereitungen bevorzugt, die, bezogen auf das Gewicht der fertigen Anwendungszubereitung, Xanthan in Mengen von 0,6 bis 5 Gew.-%, besonders bevorzugt von 1,0 bis 3,5 Gew.-% und insbesondere bevorzugt 1,5 bis 2,5 Gew.-% explizit 1,5; 1,6; 1,7; 1,8; 1,9; 2,0; 2,1; 2,2; 2,3; 2,4 oder 2,5 Gew.-%.

In einer besonderen Ausführungsform enthalten die Zubereitungen (B) gemäß der Erfindung Wasserstoffperoxid als Oxidationsmittel.

Die Konzentration einer Wasserstoffperoxid-Lösung in der Oxidationsmittelzubereitung (B) wird einerseits von den gesetzlichen Vorgaben und andererseits von dem gewünschten Effekt bestimmt. Vorzugsweise enthalten die Zubereitungen (B) bezogen auf ihr Gewicht Wasserstoffperoxid in Mengen von 0,5 bis 30 Gew.-%, bevorzugt von 1 bis 20 Gew.-%, besonders bevorzugt von 5 bis 15 Gew.-% und insbesondere bevorzugt von 6 bis 12 Gew.-%, explizit 6, 7, 8, 9, 10, 11 oder 12 Gew.-%.

Erfindungsgemäß bevorzugte anwendungsbereite Mittel sind dadurch gekennzeichnet, dass sie, bezogen auf das Gesamtgewicht des anwendungsbereiten Mittels, 0,01 bis 12 Gew.-%, vorzugsweise 0,1 bis 10 Gew.-%, besonders bevorzugt 1 bis 8 Gew.-% Wasserstoffperoxid enthalten.

Erfindungsgemäß besonders bevorzugte Mittel sind dadurch gekennzeichnet, daß die Zubereitung (B) - bezogen auf ihr Gewicht - 0,1 bis 10 Gew.-%, vorzugsweise 0,25 bis 7,5 Gew.-%, weiter bevorzugt 0,5 bis 5 Gew.-%, besonders bevorzugt 0,75 bis 4 Gew.-% und insbesondere 1 bis 2,5 Gew.-% Xanthan und 0,5 bis 30 Gew.-%, vorzugsweise 1 bis 20 Gew.-%, besonders bevorzugt 5 bis 15 Gew.-% und insbesondere 6 bis 12 Gew.-% Wasserstoffperoxid, berechnet als 100%iges H₂O₂, enthält.

Zur Stabilisierung des Wasserstoffperoxid kann der pH-Wert der Zubereitung (B) bevorzugt auf pH 3 bis 5, besonders bevorzugt auf pH 3,5 bis 4,5 und insbesondere bevorzugt auf pH 3,8 bis 4,2 eingestellt werden.

Die viskosen Eigenschaften von Zubereitung (B) sind von Bedeutung für ihre gute Mischbarkeit und hohe Stabilität. In einer bevorzugten Ausführungsform sind die Zubereitungen (B) der vorliegenden Erfindung dadurch gekennzeichnet, dass sie eine Viskosität von 1.000 mPa·s bis 50.000 mPa·s, vorzugsweise von von 5.000 mPa·s bis 45.000 mPa·s und besonders bevorzugt von 7.000 mPa·s bis 40.000 mPa·s bei Messungen mit einem Rotationsviskosimeter von Brookfield, Spindelgröße 4, bei 25°C und 4 Upm, aufweisen. Die fertig angemischten und anwendungsbereiten Mittel weisen vorzugsweise eine Viskosität von 10.000 mPa·s bis 50.000 mPa·s und besonders bevorzugt von 18.000 mPa·s bis 30.000 mPa·s bei Messungen mit einem Rotationsviskosimeter von Brookfield, Spindelgröße 5, bei 25°C und 4 Upm auf.

Weiterhin ist die Einstellung des pH-Wertes von Bedeutung für gute Mischbarkeit und Stabilität. Erfindungsgemäß bevorzugt sind fertig angemischte und anwendungsbereite Mittel, deren pH-Wert zwischen 9 und 12 liegt.

Weiterhin kann es erfindungsgemäß von Vorteil sein, wenn Zubereitung (B) mindestens ein nichtionisches Tensid, bevorzugt mindestens einen ethoxylierten Fettalkohol mit 40 bis 60 Ethylenoxideinheiten enthält. Erfindungsgemäß ist darunter ein Anlagerungsprodukt von Ethylenoxid an einen Fettalkohol zu verstehen. Fettalkohole sind dabei gesättigte und ungesättigte Alkohole mit 12 bis 24 C-Atomen, welche linear oder verzweigt sein können. Die Molmenge an Ethylenoxid, die pro Mol Fettalkohol eingesetzt wurde, bezeichnet dabei den Ethoxylierungsgrad verstanden. Als nichtionisches Tensid eignen sich dabei insbesondere Ethylenoxid-Anlagerungsprodukte an Octylalkohol (Caprylalkohol), Nonylalkohol (Pelargonylalkohol), Undecylalkohol, Undec-10-en-1-ol, Dodecylalkohol (Laurylalkohol), 2,6,8-Trimethyl-4-nonanol (Isolaurylalkohol), Tridecylalkohol, Tetradecylalkohol (Myristylalkohol), Pentadecylalkohol, Hexadecylalkohol (Cetyl-/ Palmitylalkohol), Heptadecylalkohol, Octadecylalkohol (Stearylalkohol), Isostearylalkohol, (9*Z*)-Octadec-9-en-1-ol (Oleylalkohol), (9*E*)-Octadec-9-en-1-ol (Elaidylalkohol), (9*Z*,12*Z*)-Octadeca-9,12-dien-1-ol (Linoleylalkohol), (9*Z*,12*Z*,15*Z*)-Octadeca-9,12,15-trien-1-ol (Linolenylalkohol), Nonadecan-1-ol (Nonadecylalkohol), Eicosan-1-ol (Eicosylalkohol / Arachylalkohol), (9*Z*)-Eicos-9-en-1-ol (Gadoleylalkohol), (5*Z*,8*Z*,11*Z*,14*Z*)-Eicosa-5,8,11,14-tetraen-1-ol (Arachidonalkohol), Heneicosylalkohol, Docosylalkohol (Behenylalkohol), (13Z)-Docos-13-en-1-ol (Erucylalkohol) oder (13*E*)-Docosen-1-ol (Brassidylalkohol). Es ist erfindungsgemäß ebenfalls möglich, Gemische von Fettalkoholen, die durch gezielte Mischung oder auch durch Gewinnungsverfahren als solche anfallen, einzusetzen. Beispiele sind Cocosalkohol (Mischung aus C₈-C₁₈-Fettalkoholen) oder Cetearylalkohol (1:1-Mischung aus C₁₆- und C₁₈-Fettalkoholen). Bevorzugt sind Ethoxylierungsgrade von 20 bis 60. Erfindungsgemäß bevorzugte nichtionische Tenside vom Typ ethoxylierter Fettalkohol sind Ceteareth-20 und Ceteareth-50.

Weiterhin können die Blondiermittel Alkalisierungsmittel enthalten. Bevorzugte Alkalisierungsmittel sind beispielsweise Ammoniak, Alkanolamine, basischen Aminosäuren, sowie anorganischen Alkalisierungsmittel wie (Erd-)Alkalimetallhydroxide, (Erd-)Alkalimetallmetasilikate, (Erd-) Alkalimetallphosphate und (Erd-)Alkalimetallhydrogenphosphate. Als Metallionen dienen bevorzugt Lithium, Natrium und/oder Kalium. Insbesondere bevorzugtes Alkalisierungsmittel ist Ammoniak. Erfindungsgemäß einsetzbare, anorganische Alkalisierungsmittel sind bevorzugt ausgewählt aus Natriumhydroxid, Kaliumhydroxid, Calciumhydroxid, Bariumhydroxid, Natriumphosphat, Kaliumphosphat, Natriumsilicat, Kaliumsilicat, Magnesiumsilicat, Natriumcarbonat und Kaliumcarbonat. Besonders bevorzugt sind Natriumhydroxid und/oder Kaliumhydroxid.

Erfindungsgemäß einsetzbare Alkalisierungsmittel werden bevorzugt ausgewählt aus Alkanolaminen aus primären, sekundären oder tertiären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methyl-propan-2-ol, 3-Aminopropan-1,2-diol, 2-Amino-2-methylpropan-1,3-diol, 2-Amino-2-ethyl-1,3-propandiol, N,N-Dimethyl-ethanolamin, Methylglucamin, Triethanolamin, Diethanolamin und Triisopropanolamin. Insbesondere bevorzugte Alkanolamine sind Monoethanolamin, 2-Amino-2-methyl-propanol und Triethanolamin.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D/L-Arginin, L-Lysin, D-Lysin, D/L-Lysin, L-Ornithin, D- Ornithin, D/L- Ornithin, L-Histidin, D-Histidin und/oder D/L-Histidin. Besonders bevorzugt werden L-Arginin, D-Arginin und/oder D/L-Arginin als ein Alkalisierungsmittel im Sinne der Erfindung eingesetzt.

Manche Kunden empfinden die intensive Geruchsbildung von Ammoniak belästigend oder störend. Obwohl Ammoniak ein bevorzugtes Alkalisierungsmittel ist, können daher anwendungsbereite Zubereitungen erfindungsgemäß bevorzugt sein, die frei von Ammoniak sind. Bevorzugte Alkalisierungsmittel für Zubereitungen, die frei von Ammoniak sind, sind Monoethanolamin, 2-Amino-2-methyl-propanol und Triethanolamin.

Wenn die anwendungsbereiten Mischungen Alkalisierungsmittel enthalten, sind Zubereitungen erfindungsgemäß bevorzugt, die Alkalisierungsmittel in einer Menge von 0,05 bis 20 Gew.-%, insbesondere von 0,5 bis 10 Gew.-%, jeweils bezogen auf das Gesamtgewicht des anwendungsbereiten Mittels, enthalten.

In einer weiteren Ausführungsform der Erfindung, können Zubereitungen (A) und (B) mit weiteren separat verpackten Zubereitungen unmittelbar vor der Anwendung zu einer Anwendungsmischung vermischt werden.

In einer bevorzugten Ausführungsform der Erfindung enthält das erfindungsgemäße Mittel zusätzlich mindestens eine weitere getrennt von Zubereitungen (A) und (B) verpackte Zubereitung (C), wobei Zubereitung (C) mindestens ein Alkalisierungsmittel und mindestens ein natürliches Polymer enthält.

Bevorzugt enthält Zubereitung (C) natürliche Polymere, die bereits weiter oben im Text im Zusammenhang mit Zubereitung (B) genannt wurden.

Erfindungsgemäß bevorzugt sind solche Alkalisierungsmittel, die bereits zuvor beschrieben wurden.

Wenn die Zubereitungen (C) Alkalisierungsmittel enthalten, sind Zubereitungen erfindungsgemäß bevorzugt, die Alkalisierungsmittel in einer Menge von 0,05 bis 20 Gew.-%, insbesondere von 0,5 bis 10 Gew.-%, jeweils bezogen auf das Gesamtgewicht des anwendungsbereiten Mittels, enthalten.

Unabhängig davon, ob Zubereitungen (C) und/oder Zubereitung (B) und/oder weitere Zubereitungen Alkalisierungsmittel enthalten, sind, wenn Alkalisierungsmittel zum Einsatz kommen, solche Zubereitungen erfindungsgemäß bevorzugt, die Alkalisierungsmittel in einer Menge von 0,05 bis 20 Gew.-%, insbesondere von 0,5 bis 10 Gew.-%, jeweils bezogen auf das Gesamtgewicht des anwendungsbereiten Mittels, enthalten.

Zur weiteren Steigerung der Aufhellleistung kann Zubereitung (C) zusätzlich als Bleichverstärker eine Silicium-haltige Verbindung zugesetzt werden. Diese wird bevorzugt ausgewählt aus der Gruppe, die gebildet wird aus Kieselsäure, Alkalimetallsilicaten und Erdalkalimetallsilicaten. Obwohl bereits geringe Mengen der Silicium-haltigen Verbindungen die Aufhellleistung erhöhen, kann es erfindungsgemäß bevorzugt sein, die Silicium-haltigen Verbindungen in Mengen von 0,05 Gew.-% bis 50 Gew.-%, bevorzugt in Mengen von 0,5 Gew.-% bis 30 Gew.-% und besonders bevorzugt in Mengen von 1,0 Gew.-% bis 25 Gew.-%, jeweils bezogen auf das Gesamtgewicht der Zubereitung (C), einzusetzen.

Als Silicium-haltige Verbindungen werden insbesondere Alkalimetallsilicate in Form von Wasserglas eingesetzt. Unter Wasserglas ist dabei eine Verbindung zu verstehen, die aus einem Silicat der Formel (SiO₂)ₙ(Na₂O)ₘ(K₂O)ₚ gebildet wird, wobei n für eine positive rationale Zahl und m und p unabhängig voneinander für eine positive rationale Zahl oder für 0 stehen, mit den Maßgaben, dass mindestens einer der Parameter m oder p von 0 verschieden ist und das Verhältnis zwischen n und der Summe aus m und p zwischen 1:1 und 4:1 liegt.

Neben den durch die Summenformel beschriebenen Komponenten können die Wassergläser in geringen Mengen noch weitere Zusatzstoffe, wie Phosphate oder Magnesiumsalze, enthalten. Erfindungsgemäß besonders bevorzugte Wassergläser werden unter anderem unter den Bezeichnungen Ferrosil^{®} 119, Natronwasserglas 40/42, Portil^{®} A, Portil^{®} AW und Portil^{®} W und Britesil^{®} C20 vertrieben.

Weiterhin können als Silicium-haltige Verbindungen insbesondere Kieselsäuren eingesetzt werden, die auch als Silica oder Kieselgel vertrieben werden. Bevorzugt ist dabei ein Kieselgel, welches unter dem Handelsnamen Aerosil 200 (INCI-Bezeichnung: Silica) vertrieben wird.

Weiterhin können die Aufhell- bzw. Blondiermittel zur Verstärkung der Blondierwirkung noch zusätzliche Bleichkraftverstärker enthalten. Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. i-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Als Bleichverstärker vom Typ der Kohlensäurederivate können erfindungsgemäß bevorzugt Carbonatsalze bzw. Hydrogencarbonatsalze eingesetzt werden. Diese sind bevorzugt ausgewählt aus der Gruppe der Ammonium-, Alkalimetall- (insbesondere Na und K) sowie Erdalkalimetall-(insbesondere Mg und Ca), -carbonatsalze bzw. -hydrogencarbonatsalze. Besonders bevorzugte Carbonat- bzw. Hydrogencarbonatsalze sind Ammoniumhydrogencarbonat, Ammoniumcarbonat, Natriumhydrogencarbonat, Natriumcarbonat, Kaliumhydrogencarbonat, Kaliumcarbonat, Magnesiumcarbonat und Calciumcarbonat. Diese besonders bevorzugten Salze können allein oder in deren Mischungen von mindestens zwei Vertretern als Bleichverstärker verwendet werden. Weiterhin sind Alkylcarbonate, -carbamate, Silylcarbonate sowie -carbamate geeignete Bleichverstärker.

Weiterhin erfindungsgemäß einsetzbare Bleichkraftverstärker können aus stickstoffhaltigen, gegebenenfalls kationischen Heterocyclen, insbesondere Imidazol, ausgewählt werden. Besonders bevorzugte stickstoffhaltige, heterocyclische Bleichkraftverstärker sind die quaternierten Kationen von Pyridinen und 3,4-Dihydroisochinolinen, wie Salze des 4-Acetyl-1-methylpyridiniums, insbesondere 4-Acetyl-1-methylpyridinium-p-toluolsulfonat, Salze des 2-Acetyl-1-methylpyridiniums, insbesondere 2-Acetyl-1-methylpyridinium-p-toluolsulfonat, sowie Salze des N-Methyl-3,4-dihydroisochinoliniums, insbesondere N-Methyl-3,4-dihydroisochinolinium-p-toluolsulfonat.

Weiterhin erfindungsgemäß einsetzbarer Bleichkraftverstärker ist Harnstoff.

Bleichkraftverstärker können in Zubereitung (A) und/oder Zubereitung (B) und/oder gegebenenfalls Zubereitung (C) und/oder gegebenenfalls weiteren Zubereitungen enthalten sein. Dabei können die Bleichkraftverstärker entweder in nur einer der Zubereitungen oder in zwei oder mehr der Zubereitungen enthalten sein. Hydrolyseempfindliche Bleichkraftverstärker können bevorzugt in der pulverförmigen Zubereitung (A) eingesetzt werden. Unabhängig davon, ob Bleichkraftverstärker in Zubereitung (A) und/oder Zubereitung (B) und/oder Zubereitung (C) und/oder weiteren Zubereitungen eingesetzt werden, sind diese, wenn Bleichkraftverstärker zum Einsatz kommen, bevorzugt in Mengen von 0,5 bis 30 Gew.-%, insbesondere in Mengen von 2 bis 20 Gew.-%, jeweils bezogen auf das Gesamtgewicht der fertig angemischten Blondierzubereitung, enthalten.

Weiterhin können die Aufhell- bzw. Blondiermittel zur Mattierung von unerwünschten Restfarbeindrücken, insbesondere im rötlichen oder bläulichen Bereich, bestimmte, direktziehende Farbstoffe der komplementären Farben enthalten. Dabei handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Direktziehende Farbstoffe sind als anionische, kationische und nichtionische direktziehende Farbstoffe bekannt. Die direktziehenden Farbstoffe werden jeweils bevorzugt in einer Menge von 0,001 bis 2 Gew.-%, bezogen auf die gesamte Anwendungszubereitung, eingesetzt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen. Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe. Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol. Ganz besonders bevorzugt sind Mittel, die mindestens eine Kombination aus Tetrabromphenolblau und Acid Red 92 enthalten.

Weiterhin hat es sich als vorteilhaft erweisen, wenn die anwendungsbereiten Mittel mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Diese können unterschiedlichen chemischen Gruppen angehören. Vorzugsweise werden einzeln oder im Gemisch miteinander eingesetzt. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazoliniummethochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide, insbesondere Arginin und/oder Serin; Proteinhydrolysate auf tierischer und/oder pflanzlicher Basis, wie beispielsweise Elastin-, Kollagen-, Keratin-, Seiden- und Milcheiweiß-Proteinhydrolysate, oder Mandel-, Reis-, Erbsen-, Kartoffel- und Weizenproteinhydrolysate, sowie in Form ihrer Fettsäure-Kondensationsprodukte oder gegebenenfalls anionisch oder kationisch modifizierten Derivate; Lichtschutzmittel, wie derivatisierte Benzophenone, Zimtsäure-Derivate und Triazine; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen, insbesondere der Gruppen A, B₃, B₅, B₆, C, E, F und H; Pflanzenextrakte; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Besonders bevorzugt gemäß der Erfindung sind solche Wirk-, Hilfs- und Zusatzstoffe, die in Kombination mit dem erfindungsgemäßen Mittel zu einer transparenten Anwendungsmischung führen.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, bezogen auf das Gesamtgewicht der Anwendungsmischung, eingesetzt.

Ein zweiter Gegenstand der Erfindung ist ein Verfahren zur Farbveränderung keratinischer Fasern, bei dem mindestens zwei getrennt voneinander verpackte Zubereitungen (A) und (B), von welchen Zubereitung (A) mindestens ein Persulfat und Zubereitung (B) mindestens ein Oxidationsmittel enthält, zu einer Anwendungsmischung vermischt werden, diese auf die Fasern aufgetragen wird und nach einer Einwirkungszeit wieder abgespült wird, dadurch gekennzeichnet, dass
i. Zubereitung (B) und/oder eine ggf. vorhandene Zubereitung (C) mindestens ein natürliches Polymer enthält/enthalten,
ii. Zubereitung (A) - bezogen auf ihr Gewicht -
   a1) 0 bis 3 Gew.-% Keratinhydrolysat(e) und/oder
   a2) 0 bis 5 Gew.-% Silikonöl(e) enthält,
   mit der Maßgabe, dass die Zubereitung (A) - bezogen auf ihr Gewicht - 0,1 bis 6 Gew.-% Verbindung(en) aus den Gruppen a1) und a2) enthält,
   wobei die Zubereitung (A) 0,01 bis 1 Gew.-% Hydrolysat(e) mit Molmassen von 400 bis 1200 Dalton, gewonnen aus dem Kortex und/oder der Cuticula von keratinischen Fasern, enthält.

Die anwendungsbereiten Mittel werden unmittelbar vor der Anwendung auf dem Haar durch Mischen der zwei Zubereitungen (A) und (B) und gegebenenfalls einer dritten Zubereitung (C) und/oder weiteren Zubereitungen hergestellt. Bei anwendungsbereiten Mitteln, die aus mehr als zwei Zubereitungen zu einer fertigen Anwendungsmischung vermischt werden, kann es unerheblich sein, ob zunächst zwei Zubereitungen miteinander vermischt werden und anschließend die dritte Zubereitung zugegeben und untergemischt wird, oder ob alle Zubereitungen gemeinsam zusammengeführt und anschließend vermischt werden. Das Vermischen kann durch Verrühren in einer Schale oder einem Becher erfolgen oder durch Schütteln in einem verschließbaren Behälter.

Der Begriff "unmittelbar" ist dabei als Zeitraum von wenigen Sekunden bis eine Stunde, vorzugsweise bis 30 min, insbesondere bis 15 min zu verstehen.

Die erfindungsgemäßen Mittel werden in einem Verfahren zum Aufhellen von keratinischen Fasern, insbesondere menschlichen Haaren, angewendet, bei dem das Mittel auf die keratinhaltigen Fasern aufgebracht, bei einer Temperatur von Raumtemperatur bis 45 °C für eine Einwirkdauer von 10 bis 60 Minuten auf der Faser belassen und anschließend mit Wasser wieder ausgespült oder mit einem Shampoo ausgewaschen wird.

Bevorzugt beträgt die Einwirkzeit der anwendungsbereiten Aufhellmittel 10 bis 60 min, insbesondere 15 bis 50 min, besonders bevorzugt 20 bis 45 min. Während der Einwirkzeit des Mittels auf der Faser kann es vorteilhaft sein, den Aufhellvorgang durch Wärmezufuhr zu unterstützen. Die Wärmezufuhr kann durch eine externe Wärmequelle, wie mit Hilfe eines Warmluftgebläses, als auch, insbesondere bei einer Haaraufhellung am lebenden Probanden, durch die Körpertemperatur des Probanden erfolgen. Bei letzterer Möglichkeit wird üblicherweise die aufzuhellende Partie mit einer Haube abgedeckt. Eine Einwirkphase bei Raumtemperatur ist ebenfalls erfindungsgemäß. Bevorzugt liegt die Temperatur während der Einwirkzeit zwischen 20°C und 40°C, insbesondere zwischen 25°C und 38°C. Die Aufhellmittel ergeben bereits bei physiologisch verträglichen Temperaturen von unter 45°C gute Blondier- und Aufhellergebnisse.

Nach Ende der Einwirkzeit wird die verbleibende Aufhellzubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Leitungswasser erfolgen kann, wenn das Aufhellmittel einen stark tensid-haltigen Träger besitzt.

Ein bevorzugtes Verfahren ist dadurch gekennzeichnet, dass die Kontrolle des Aufhellgrades der keratinischen Faser während der Einwirkzeit ohne Entfernen der Anwendungsmischung von der Faser visuell erfolgt. Dazu wird ein anwendungsbereites, transparentes Mittel des ersten Erfindungsgegenstands auf menschliches Haar aufgetragen, und der Aufhellprozess während der Einwirkzeit ein oder mehrmals durch visuelle Kontrolle, ohne Abtragen des Mittels von der Faser, beurteilt. Somit ist eine vereinfachte und permanente Kontrolle des Entfärbevorgangs gewährleistet.

"Transparent" im Sinne der Erfindung sind Zubereitungen, die bei Auftragung einer gleichmäßigen Schicht dieser Zubereitungen von 1 bis 3 mm Dicke auf einen Untergrund klar sind und so durchblickt werden können, dass das menschliche Auge die Farbe des Untergrunds ohne Trübung erkennen und beurteilen kann. Transparenz kann vom Fachmann weiterhin durch technische Methoden gemessen werden. "Transparent" im Sinne der Erfindung sind daher auch Zubereitungen, die bei photometrischen Messungen mit einem Methrom Photometer 662 bei 25°C Transm issionen von mindestens 70%, insbesondere mindestens 80%, erreichen.

Erfindungsgemäß bevorzugte Verfahren sind dadurch gekennzeichnet, daß die Kontrolle des Aufhellgrades der keratinischen Faser während der Einwirkzeit ohne Entfernen der Anwendungsmischung von der Faser visuell erfolgt.

Die bevorzugten Ausführungsformen des ersten Erfindungsgegenstands gelten mutatis mutandis auch für den zweiten Erfindungsgegenstand.

Ein dritter Gegenstand der vorliegenden Erfindung ist die Verwendung von Keratinhydrolysaten zur Steigerung der Aufhell- und Pflegeleistung transparenter Blondiermittel. Bezüglich bevorzugter Ausführungsformen der erfindungsgemäßen Verwendungen gelten mutatis mutandis die Ausführungsformen der vorangegangenen Erfindungsgegenstände.

### Beispiele

**Beispiel 1: Zusammensetzung Zubereitung (A) für Blondiermittel aus zwei Zubereitungen**

| Beschreibung | A1 | A2 | A3 | A4 | A5 ⁺ | A6 ⁺ | A7 ⁺ | A8 |
|---|---|---|---|---|---|---|---|---|
| EDTA Na2 | 1,51 | 1 | 1,51 | 1,51 | 1,51 | 1,51 | 1,51 | 1,51 |
| Magnesiumcarbonat | 8,0 | 7,5 | 7,0 | 8,5 | 9,0 | 8,0 | 8,0 | 8,0 |
| Aerosil 200 | 3,02 | 1 | 3 | 3 | 3 | 3 | 3 | 3 |
| Natriummetasilikat FE wasserfrei | 4,7 | 5,0 | 5,5 | 4,0 | 4,5 | 4,7 | 4,7 | 4,7 |
| Natriumdisilikat | 31,05 | 30,0 | 31,0 | 30,5 | 31,5 | 31,05 | 31,0 | 31,0 |
| Rohagit S hv | 2,75 | 4,05 | 1 | | | 2,5 | 3,5 | |
| Cekol 50000 | 1,1 | 1,0 | 1,5 | 1,0 | 1,1 | 1,1 | | |
| Ammoniumpersulfat + 0,5 % Kieselsäure | 10,0 | 10,0 | 10,0 | 10,0 | 10,0 | 10,0 | 10,0 | 10,0 |
| Kaliumpersulfat | 30,0 | 30,0 | 30,0 | 30,0 | 30,0 | 30,0 | 30,0 | 30,0 |

| | | | | | | | | |
|---|---|---|---|---|---|---|---|---|
| ⁺ = Referenzbeispiel | | | | | | | | |

**Beispiel 2: Zusammensetzung Zubereitung (B) für Blondiermittel aus zwei Zubereitungen**

| Beschreibung | A1 | A2 | A3 | A4 | A5 ⁺ | A6 ⁺ | A7 ⁺ | A8 |
|---|---|---|---|---|---|---|---|---|
| Natriumpersulfat | 4,0 | 4,0 | 4,0 | 4,0 | 4,0 | 4,0 | 4,0 | 4,0 |
| Paraffinum Liquidum | 3,6 | 3,6 | 3,6 | 3,6 | 3,6 | 3,6 | 3,6 | 3,6 |
| Parfum | 0,2 | 0,2 | 0,2 | 0,2 | 0,2 | 0,2 | 0,2 | 0,2 |
| Dimethicone, 60.000 cSt | 0,2 | 0,1 | | | 0,1 | 0,15 | 0,2 | 0,2 |
| Keratinhydrolysat, pulverförmig | 1,5 | 1,5 | 2,0 | 1,5 | | | | 1,5 |
| *eingesetzte Rohstoffe: Aerosil 200 (INCI Bezeichnung: Silica (Evonik Degussa)), Rohagit S hv (INCI Bezeichnung: Acrylates Copolymer (Evonik Röhm)), Cekol 50000 (INCI Bezeichnung: Cellulose Gummi (CP Kelco)), LAPONITE XLG (INCI Bezeichnung: Natrium-Magnesium-Silicat (Roockwood Additives)), LAPONITE XLS (INCI Bezeichnung: Natrium-Magnesium-Silicat und Tetranatriumpyrophosphat (Roockwood Additives)), | | | | | | | | |

| Beschreibung | B1 | B2 | B3 | B4 | B5 |
|---|---|---|---|---|---|
| Wasser, vollentsalzt | ad 100 | ad 100 | ad 100 | ad 100 | ad 100 |
| Natronlauge 45 % techn. | 0,73 | 0,73 | 0,73 | 0,73 | 0,73 |
| Dipicolinsäure | 0,1 | 0,1 | 0,1 | 0,1 | 0,1 |
| Dinatrium pyrophosphat | 0,03 | 0,03 | 0,03 | 0,03 | 0,03 |
| HEDP 60% | 1,5 | 1,5 | 1,5 | 1,5 | 1,5 |
| Keltrol CG-SFT | 2 | | 1,5 | 1 | 0,5 |
| Propandiol-1,2 | 4 | 2 | 4 | 4 | 4 |
| Wasserstoffperoxid 50 % | 18 | 18 | 18 | 18 | 18 |

| | | | | | |
|---|---|---|---|---|---|
| *eingesetzte Rohstoffe: Keltrol CG-SFT (INCI Bezeichnung: Xanthan Gum (CP Kelco)), Bei den Zubereitungen B1 bis B5 handelt es sich um transparente Gele. | | | | | |

## Patentansprüche

1. Mittel zum Aufhellen keratinischer Fasern, enthaltend mindestens zwei getrennt voneinander verpackte Zubereitungen (A) und (B) sowie gegebenenfalls eine weitere getrennt von (A) und (B) verpackte Zubereitung (C), die unmittelbar vor der Anwendung zu einer Anwendungsmischung vermischt werden, wobei
iii. Zubereitung (A) mindestens ein Persulfat enthält und
iv. Zubereitung (B) fließfähig ist und mindestens ein Oxidationsmittel enthält,
v. Zubereitung (B) und/oder Zubereitung (C) weiterhin mindestens ein natürliches Polymer enthält/enthalten,
**dadurch gekennzeichnet, dass** Zubereitung (A) - bezogen auf ihr Gewicht -
a1) 0 bis 3 Gew.-% Keratinhydrolysat(e) und/oder
a2) 0 bis 5 Gew.-% Silikonöl(e) enthält,
mit der Maßgabe, daß die Zubereitung (A) - bezogen auf ihr Gewicht - 0,1 bis 6 Gew. % Verbindung(en) aus den Gruppen a1) und a2) enthält;
wobei die Zubereitung (A) 0,01 bis 1 Gew.-% Hydrolysat(e) mit Molmassen von 400 bis 1200 Dalton, gewonnen aus dem Kortex und/oder der Cuticula von keratinischen Fasern, enthält.

2. Mittel nach Anspruch 1, **dadurch gekennzeichnet, dass** die Zubereitung (A) 0,05 bis 0,75 Gew.-%, bevorzugt 0,1 bis 0,5 Gew.-%, besonders bevorzugt 0,15 bis 0,4 Gew.-% und insbesondere 0,2 bis 0,3 Gew.-% Hydrolysat(e) mit Molmassen von 400 bis 1200 Dalton, gewonnen aus dem Kortex und/oder der Cuticula von keratinischen Fasern, enthält.

3. Mittel nach einem der Ansprüche 1 oder 2, **dadurch gekennzeichnet, daß** die Zubereitung (A) 0,01 bis 5 Gew.-%, vorzugsweise 0,05 bis 4 Gew.-%, weiter bevorzugt 0,1 bis 2,5 Gew.-%, besonders bevorzugt 0,25 bis 2 Gew.-% und insbesondere 0,5 bis 1,5 Gew.-% Silikonöl(e) aus der Gruppe der Verbindungen mit der INCI-Bezeichnung Dimethicone enthält.

4. Mittel nach einem der Ansprüche 1 bis 3, **dadurch gekennzeichnet, dass** die Zubereitung (A) 0,01 bis 5 Gew.-%, vorzugsweise 0,05 bis 4 Gew.-%, weiter bevorzugt 0,1 bis 2,5 Gew.-%, besonders bevorzugt 0,25 bis 2 Gew.-% und insbesondere 0,5 bis 1,5 Gew.-% Silikonöl(e) aus der Gruppe der Verbindungen mit der INCI-Bezeichnung Amodimethicone enthält, wobei Verbindungen mit den INCI-Bezeichnungen Silicone Quaternium-1, Silicone Quaternium-2, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, Silicone Quaternium-16, Silicone Quaternium-17, Silicone Quaternium-18, Silicone Quaternium-20, Silicone Quaternium-21 oder Silicone Quaternium-22 bevorzugt sind.

5. Mittel nach einem der Ansprüche 1 bis 4, **dadurch gekennzeichnet, dass** die Zubereitung (A) 0,01 bis 5 Gew.-%, vorzugsweise 0,05 bis 4 Gew.-%, weiter bevorzugt 0,1 bis 2,5 Gew.-%, besonders bevorzugt 0,25 bis 2 Gew.-% und insbesondere 0,5 bis 1,5 Gew.-% Silikonöl(e) der Formel (I) enthält, 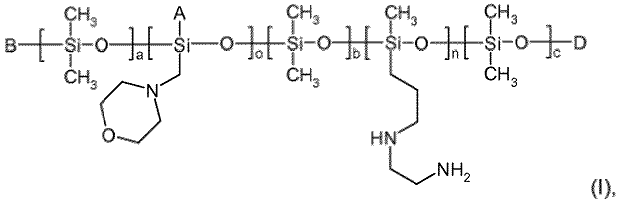 in der
A für eine über ein -O- gebundene Struktureinheit (I), (II) oder (III) 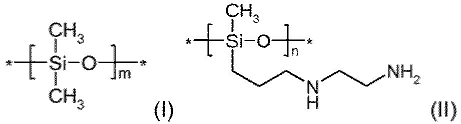 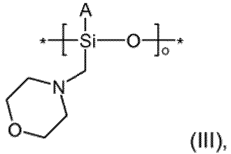
oder einen über ein -O- gebundenen oligomeren oder polymeren Rest enthaltend Struktureinheiten der Formeln (I), (II) oder (III) oder die Hälfte eines verbindenden O-Atoms zu einer Struktureinheit (III) oder für -OH steht,
* für eine Bindung zu einer der Struktureinheiten (I), (II) oder (III) oder für eine Endgruppe B (Si-gebunden) oder D (O-gebunden) steht,
B für eine Gruppe -OH, -O-Si(CH₃)₃, -O-Si(CH₃)₂OH, -O-Si(CH₃)₂OCH₃ steht,
D für eine Gruppe -H, -Si(CH₃)₃, -Si(CH₃)₂OH, -Si(CH₃)₂OCH₃ steht,
a, b und c für ganze Zahlen zwischen 0 und 1000 stehen, mit der Maßgabe a + b + c > 0
m, n und o für ganze Zahlen zwischen 1 und 1000 stehen.

6. Mittel nach einem der Ansprüche 1 bis 5, **dadurch gekennzeichnet, dass** Zubereitung (A) frei von Xanthan ist und - bezogen auf ihr Gewicht - 5 bis 60 Gew.-%, vorzugsweise 10 bis 55 Gew.-%, besonders bevorzugt 15 bis 50 Gew.-% und insbesondere 20 bis 45 Gew.-% mindestens eines Persulfat, ausgewählt aus Ammoniumperoxodisulfat und/oder Kaliumperoxodisulfat und/oder Natriumperoxodisulfat, enthält.

7. Mittel nach einem der Ansprüche 1 bis 6, **dadurch gekennzeichnet, dass** Zubereitung (A) Persulfat-Salze in einer Menge von 0,1 bis 80 Gew.-%, bevorzugt von 2 bis 50 Gew.-%, besonders bevorzugt von 3 bis 30 Gew.-% und insbesondere bevorzugt 5 bis 15 Gew.-% explizit 5, 6, 7, 8, 9, 10, 11, 12, 13, 14 oder 15 Gew.-%, jeweils bezogen auf das Gesamtgewicht des Mittels, enthält.

8. Mittel nach einem der Ansprüche 1 bis 7, **dadurch gekennzeichnet, dass** die Zubereitung (B) - bezogen auf ihr Gewicht - 0,1 bis 10 Gew.-%, vorzugsweise 0,25 bis 7,5 Gew.-%, weiter bevorzugt 0,5 bis 5 Gew.-%, besonders bevorzugt 0,75 bis 4 Gew.-% und insbesondere 1 bis 2,5 Gew.-% Xanthan und 0,5 bis 30 Gew.-%, vorzugsweise 1 bis 20 Gew.-%, besonders bevorzugt 5 bis 15 Gew.-% und insbesondere 6 bis 12 Gew.-% Wasserstoffperoxid, berechnet als 100%iges H₂O₂, enthält.

9. Mittel nach einem der Ansprüche 1 bis 8, **dadurch gekennzeichnet, dass** die Zubereitung (B) einen pH-Wert von 3 bis 5, besonders bevorzugt von pH 3,5 bis 4,5 und insbesondere bevorzugt von pH 3,8 bis 4,2 aufweist.

10. Mittel nach einem der Ansprüche 1 bis 9, **dadurch gekennzeichnet, dass** die Zubereitung (B) eine Viskosität von 1.000 mPa·s bis 50.000 mPa·s, vorzugsweise von von 5.000 mPa·s bis 45.000 mPa·s und besonders bevorzugt von 7.000 mPa·s bis 40.000 mPa·s bei Messungen mit einem Rotationsviskosimeter von Brookfield, Spindelgröße 4, bei 25°C und 4 Upm, aufweist.

11. Mittel nach einem der Ansprüche 1 bis 10, **dadurch gekennzeichnet, dass** die fertig angemischten und anwendungsbereiten Mittel eine Viskosität von 10.000 mPa·s bis 50.000 mPa·s und besonders bevorzugt von 18.000 mPa·s bis 30.000 mPa·s bei Messungen mit einem Rotationsviskosimeter von Brookfield, Spindelgröße 5, bei 25°C und 4 Upm aufweisen.

12. Mittel nach einem der Ansprüche 1 bis 11, **dadurch gekennzeichnet, dass** die fertig angemischten und anwendungsbereiten Mittel einen pH-Wert zwischen 9 und 12 aufweisen.

13. Verfahren zur Farbveränderung keratinischer Fasern, bei dem mindestens zwei getrennt voneinander verpackte Zubereitungen (A) und (B), von welchen Zubereitung (A) mindestens ein Persulfat und Zubereitung (B) mindestens ein Oxidationsmittel enthält, zu einer Anwendungsmischung vermischt werden, diese auf die Fasern aufgetragen wird und nach einer Einwirkungszeit wieder abgespült wird, **dadurch gekennzeichnet, dass**
i. Zubereitung (B) und/oder eine ggf. vorhandene Zubereitung (C) mindestens ein natürliches Polymer enthält/enthalten,
ii. Zubereitung (A) - bezogen auf ihr Gewicht -
a1) 0 bis 3 Gew.-% Keratinhydrolysat(e) und/oder
a2) 0 bis 5 Gew.-% Silikonöl(e) enthält,
mit der Maßgabe, dass die Zubereitung (A) - bezogen auf ihr Gewicht - 0,1 bis 6 Gew.-% Verbindung(en) aus den Gruppen a1) und a2) enthält,
wobei die Zubereitung (A) 0,01 bis 1 Gew.-% Hydrolysat(e) mit Molmassen von 400 bis 1200 Dalton, gewonnen aus dem Kortex und/oder der Cuticula von keratinischen Fasern, enthält.

14. Verfahren nach Anspruch 13, **dadurch gekennzeichnet, dass** die Kontrolle des Aufhellgrades der keratinischen Faser während der Einwirkzeit ohne Entfernen der Anwendungsmischung von der Faser visuell erfolgt.

15. Verwendung von Keratinhydrolysaten zur Steigerung der Aufhell- und Pflegeleistung transparenter Blondiermittel.

## Claims

1. An agent for lightening keratin fibers, containing at least two separately packaged preparations (A) and (B) and optionally a further preparation (C) that is packaged separately from (A) and (B), which preparations are mixed immediately before application to form an application mixture,
iii. preparation (A) containing at least one persulfate, and
iv. preparation (B) being flowable and containing at least one oxidizing agent,
v. preparation (B) and/or preparation (C) also containing at least one natural polymer, **characterized in that** preparation (A) contains, based on its weight,
a1) from 0 to 3 wt.% of keratin hydrolyzate(s), and/or
a2) from 0 to 5 wt.% of silicone oil(s),
with the proviso that preparation (A) contains, based on its weight, from 0.1 to 6 wt.% of compound(s) from groups a1) and a2),
wherein preparation (A) contains from 0.01 to 1 wt.% of hydrolyzate(s) that have molar masses of from 400 to 1200 daltons and are obtained from the cortex and/or cuticle of keratin fibers.

2. The agent according to claim 1, **characterized in that** preparation (A) contains from 0.05 to 0.75 wt.%, preferably from 0.1 to 0.5 wt.%, particularly preferably from 0.15 to 0.4 wt.%, and in particular from 0.2 to 0.3 wt.%, of hydrolyzate(s) that have molar masses of from 400 to 1200 daltons and are obtained from the cortex and/or cuticle of keratin fibers.

3. The agent according to one of claims 1 or 2, **characterized in that** preparation (A) contains from 0.01 to 5 wt.%, preferably from 0.05 to 4 wt.%, more preferably from 0.1 to 2.5 wt.%, particularly preferably from 0.25 to 2 wt.%, and in particular from 0.5 to 1.5 wt.%, of silicone oil(s) from the group of compounds having the INCI name dimethicone.

4. The agent according to one of claims 1 to 3, **characterized in that** preparation (A) contains from 0.01 to 5 wt.%, preferably from 0.05 to 4 wt.%, more preferably from 0.1 to 2.5 wt.%, particularly preferably from 0.25 to 2 wt.%, and in particular from 0.5 to 1.5 wt.%, of silicone oil(s) from the group of compounds having the INCI name amodimethicone, compounds having the INCI names Silicone Quaternium-1, Silicone Quaternium-2, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, Silicone Quaternium-16, Silicone Quaternium-17, Silicone Quaternium-18, Silicone Quaternium-20, Silicone Quaternium-21 or Silicone Quaternium-22 being preferred.

5. The agent according to one of claims 1 to 4, **characterized in that** preparation (A) contains from 0.01 to 5 wt.%, preferably from 0.05 to 4 wt.%, more preferably from 0.1 to 2.5 wt.%, particularly preferably from 0.25 to 2 wt.%, and in particular from 0.5 to 1.5 wt.%, of silicone oil(s) of formula (I), 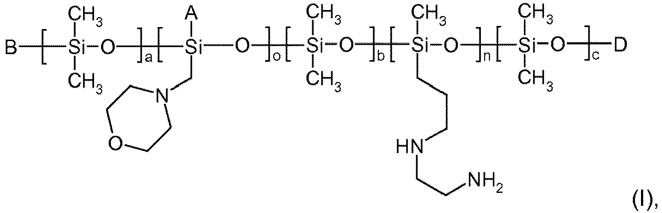 in which
A represents a structural unit (I), (II) or (III) bound by an -O- 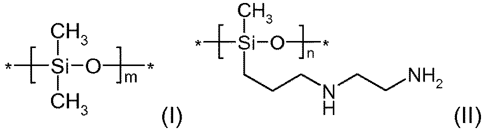 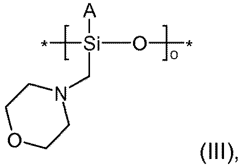
or an oligomeric or polymeric functional group bound by an -O- and containing structural units of formulas (I), (II) or (III), or one half of an O atom binding to a structural unit (III), or represents -OH,
* represents a bond to one of the structural units (I), (II) or (III) or represents a terminal group B (Si-bound) or D (O-bound),
B represents a -OH, -O-Si(CH₃)₃ , -O-Si(CH₃)₂OH, -O-Si(CH₃)₂ OCH₃ group,
D represents a -H, -Si(CH₃)₃ , -Si(CH₃)₂ OH, -Si(CH₃)₂ OCH₃ group, a, b and c represent integers between 0 and 1000, with the proviso that a + b + c > 0 m, n and o represent integers between 1 and 1000.

6. The agent according to one of claims 1 to 5, **characterized in that** preparation (A) is free of xanthan gum and contains, based on its weight, from 5 to 60 wt.%, preferably from 10 to 55 wt.%, particularly preferably from 15 to 50 wt.%, and in particular from 20 to 45 wt.%, of at least one persulfate, selected from ammonium peroxydisulfate and/or potassium peroxydisulfate and/or sodium peroxydisulfate.

7. The agent according to one of claims 1 to 6, **characterized in that** preparation (A) contains persulfate salts in an amount of from 0.1 to 80 wt.%, preferably from 2 to 50 wt.%, particularly preferably from 3 to 30 wt.%, and in particular from 5 to 15 wt.%, explicitly 5, 6, 7, 8, 9, 10, 11, 12, 13, 14 or 15 wt.%, based in each case on the total weight of the agent.

8. The agent according to one of claims 1 to 7, **characterized in that** preparation (B) contains, based on its weight, from 0.1 to 10 wt.%, preferably from 0.25 to 7.5 wt.%, more preferably from 0.5 to 5 wt.%, particularly preferably from 0.75 to 4 wt.%, and in particular from 1 to 2.5 wt.%, of xanthan gum and from 0.5 to 30 wt.%, preferably from 1 to 20 wt.%, particularly preferably from 5 to 15 wt.%, and in particular from 6 to 12 wt.%, of hydrogen peroxide, calculated as 100% H₂O₂.

9. The agent according to one of claims 1 to 8, **characterized in that** preparation (B) has a pH of from 3 to 5, particularly preferably from 3.5 to 4.5, and more particularly preferably from 3.8 to 4.2.

10. The agent according to one of claims 1 to 9, **characterized in that** preparation (B) has a viscosity of from 1,000 mPa·s to 50,000 mPa·s, preferably from 5,000 mPa·s to 45,000 mPa·s, and particularly preferably from 7,000 mPa·s to 40,000 mPa·s, in measurements using a Brookfield rotational viscometer, spindle size 4, at 25 °C and 4 rpm.

11. The agent acording to one of claims 1 to 10, **characterized in that** the finished, mixed and ready-to-apply agents have a viscosity of from 10,000 mPa·s to 50,000 mPa·s and particularly preferably from 18,000 mPa·s to 30,000 mPa·s, in measurements using a Brookfield rotational viscometer, spindle size 5, at 25 °C and 4 rpm.

12. The agent according to one of claims 1 to 11, **characterized in that** the finished, mixed and ready-to-apply agents have a pH of between 9 and 12.

13. A method for changing the color of keratin fibers, in which at least two separately packaged preparations (A) and (B), of which preparation (A) contains at least one persulfate and preparation (B) contains at least one oxidizing agent, are mixed to form an application mixture, which is applied to the fibers and after a contact time is rinsed off again, **characterized in that**
i. preparation (B) and/or an optionally present preparation (C) contain(s) at least one natural polymer,
ii. preparation (A) contains, based on its weight,
a1) from 0 to 3 wt.% of keratin hydrolyzate(s), and/or
a2) from 0 to 5 wt.% of silicone oil(s),
with the proviso that preparation (A) contains, based on its weight, from 0.1 to 6 wt.% of compound(s) from groups a1) and a2),
wherein preparation (A) contains from 0.01 to 1 wt.% of hydrolyzate(s) that have molar masses of from 400 to 1200 daltons and are obtained from the cortex and/or cuticle of keratin fibers.

14. The method according to claim 13, **characterized in that** monitoring the degree of lightening of the keratin fiber during the contact time takes place visually without removing the application mixture from the fiber.

15. The use of keratin hydrolyzates for increasing the lightening and nourishing performance of a transparent blonding agent.

## Revendications

1. Agent d'éclaircissement de fibres de kératine, contenant au moins deux préparations (A) et (B) emballées séparément et éventuellement une autre préparation (C) emballée séparément de (A) et de (B), lesquelles préparations sont mélangées immédiatement avant l'application pour obtenir un mélange d'application,
iii. la préparation (A) contenant au moins un persulfate et
iv. la préparation (B) étant à écoulement libre et contenant au moins un agent oxydant,
v. la préparation (B) et/ou la préparation (C) contenant en outre au moins un polymère naturel,
**caractérisé en ce que** la préparation (A) contient, sur la base de son poids,
a1) de 0 à 3 % en poids d'au moins un hydrolysat de kératine et/ou
a2) de 0 à 5 % en poids d'au moins une huile de silicone,
à condition que la préparation (A) contienne, sur la base de son poids, de 0,1 à 6 % en poids d'au moins un composé des groupes a1) et a2),
la préparation (A) contenant de 0,01 à 1 % en poids d'au moins un hydrolysat dont le poids moléculaire va de 400 à 1200 daltons, obtenu à partir du cortex et/ou de la cuticule de fibres de kératine.

2. Agent selon la revendication 1, **caractérisé en ce que** la préparation (A) contient de 0,05 à 0,75 % en poids, de préférence de 0,1 à 0,5 % en poids, de manière particulièrement préférée de 0,15 à 0,4 % en poids et en particulier de 0,2 à 0,3 % d'au moins un hydrolysat dont le poids moléculaire va de 400 à 1200 daltons, obtenu à partir du cortex et/ou de la cuticule de fibres de kératine.

3. Agent selon l'une des revendications 1 ou 2, **caractérisé en ce que** la préparation (A) contient de 0,01 à 5 % en poids, de préférence de 0,05 à 4 % en poids, plus préférablement de 0,1 à 2,5 % en poids, de manière particulièrement préférée de 0,25 à 2 % en poids et en particulier de 0,5 à 1,5 % en poids d'au moins une huile de silicone du groupe de composés ayant la désignation INCI Dimethicone.

4. Agent selon l'une des revendications 1 à 3, **caractérisé en ce que** la préparation (A) contient de 0,01 à 5 % en poids, de préférence de 0,05 à 4 % en poids, plus préférablement de 0,1 à 2,5 % en poids, de manière particulièrement préférée de 0,25 à 2 % en poids et en particulier de 0,5 à 1,5 % en poids d'au moins une huile de silicone du groupe de composés ayant la désignation INCI Amodimethicone, les composés dont les désignations INCI sont Silicone Quaternium-1, Silicone Quaternium-2, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, Silicone Quaternium-16, Silicone Quaternium-17, Silicone Quaternium-18, Silicone Quaternium-20, Silicone Quaternium-21 ou Silicone Quaternium-22 étant préférés.

5. Agent selon l'une quelconque des revendications 1 à 4, **caractérisé en ce que** la préparation (A) contient de 0,01 à 5 % en poids, de préférence de 0,05 à 4 % en poids, plus préférablement de 0,1 à 2,5 % en poids, de manière particulièrement préférée de 0,25 à 2 % en poids et en particulier de 0,5 à 1,5 % en poids d'au moins une huile de silicone de la formule (I), 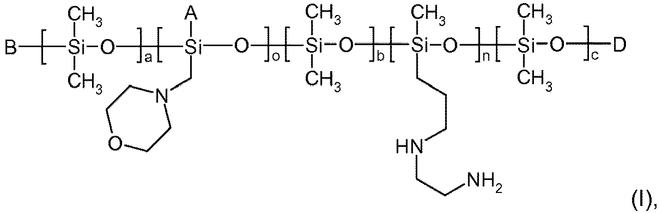 dans laquelle
A représente une unité structurelle (I), (II) ou (III) liée par un groupe -O- 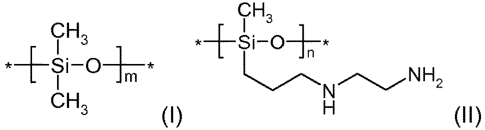 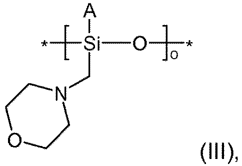
ou un radical oligomère ou polymère, lié un groupe -O-, qui contient des unités structurelles des formules (I), (II) ou (III) ou la moitié d'un atome O de liaison à une unité structurelle (III) ou représente -OH ;
* représente une liaison à l'une des unités structurelles (I), (II) ou (III) ou représente un groupe terminal B (lié par Si) ou D (lié par O),
B représente un groupe -OH, -O-Si(CH₃)₃ , -O-Si(CH₃)₂OH, -O-Si(CH₃)₂ OCH₃,
D représente un groupe -H, -Si(CH₃)₃ , -Si(CH₃)₂ OH, -Si(CH₃)₂ OCH₃,
a, b et c sont des nombres entiers compris entre 0 et 1000, avec la condition que a + b + c > 0
m, n et o sont des nombres entiers compris entre 1 et 1000.

6. Agent selon l'une des revendications 1 à 5, **caractérisé en ce que** la préparation (A) est dépourvue de xanthane et contient, sur la base de son poids, de 5 à 60 % en poids, de préférence de 10 à 55 % en poids, plus préférablement de 15 à 50 % en poids et en particulier de 20 à 45 % en poids, d'au moins un persulfate choisi parmi le peroxodisulfate d'ammonium et/ou le peroxodisulfate de potassium et/ou le peroxodisulfate de sodium.

7. Agent selon l'une des revendications 1 à 6, **caractérisé en ce que** la préparation (A) contient des sels de persulfate dans une quantité de 0,1 à 80 % en poids, de préférence de 2 % à 50 % en poids, de manière particulièrement préférée de 3 à 30 % en poids et notamment de préférence de 5 à 15 % en poids explicitement de 5, 6, 7, 8, 9, 10, 11, 12, 13, 14 ou 15 % en poids, à chaque fois sur la base du poids total de l'agent.

8. Agent selon l'une des revendications 1 à 7, **caractérisé en ce que** la préparation (B) contient, sur la base de son poids, de 0,1 à 10 % en poids, de préférence de 0,25 à 7,5 % en poids, plus préférablement de 0,5 à 5 % en poids, de manière particulièrement préférée de 0,75 à 4 % en poids et en particulier de 1 à 2,5 % en poids de xanthane et de 0,5 à 30 % en poids, de préférence de 1 à 20 % en poids, plus préférablement de 5 à 15 % en poids et en particulier de 6 à 12 % en poids de peroxyde d'hydrogène, calculé comme 100 % de H₂O₂.

9. Agent selon l'une des revendications 1 à 8, **caractérisé en ce que** la préparation (B) a une valeur de pH allant de 3 à 5, plus préférablement de 3,5 à 4,5 et de manière particulièrement préférée de 3,8 à 4,2.

10. Agent selon l'une des revendications 1 à 9, **caractérisé en ce que** la préparation (B) a une viscosité de 1000 mPa·s à 50 000 mPa·s, de préférence de 5000 mPa·s à 45 000 mPa·s et de manière particulièrement préférée de 7000 mPa·s à 40 000 mPa·s, mesurée avec un viscosimètre rotatif Brookfield, taille de broche 4, à 25 °C et 4 tr/min.

11. Agent selon l'une des revendications 1 à 10, **caractérisé en ce que** l'agent déjà mélangé et prêt à l'emploi a une viscosité de 10 000 mPa·s à 50 000 mPa·s et de manière particulièrement préférée de 18 000 mPa·s à 30 000 mPa·s, mesurée avec un viscosimètre rotatif Brookfield, taille de broche 5, à 25 °C et 4 tr/min.

12. Agent selon l'une des revendications 1 à 11, **caractérisé en ce que** l'agent déjà mélangé et prêt à l'emploi a un pH compris entre 9 et 12.

13. Procédé de modification de la couleur des fibres de kératine, dans lequel au moins deux préparations (A) et (B) emballées séparément, dont la préparation (A) contient au moins un persulfate et la préparation (B) contient au moins un agent oxydant, sont mélangées pour obtenir un mélange d'application, celui-ci est appliqué sur les fibres et éliminé par rinçage après un temps d'action, **caractérisé en ce que**
i. la préparation (B) et/ou une préparation (C) éventuellement présente contiennent au moins un polymère naturel,
ii. la préparation (A) contient, sur la base de son poids,
a1) de 0 à 3 % en poids d'au moins un hydrolysat de kératine et/ou
a2) de 0 à 5 % en poids d'au moins une huile de silicone,
avec la condition que la préparation (A) contienne, sur la base de son poids, de 0,1 à 6 % en poids d'au moins un composé des groupes a1) et a2),
la préparation (A) contenant de 0,01 à 1 % en poids d'au moins un hydrolysat dont le poids moléculaire va de 400 à 1200 daltons, obtenu à partir du cortex et/ou de la cuticule de fibres de kératine.

14. Procédé selon la revendication 13, **caractérisé en ce que** le contrôle du degré d'éclaircissement de la fibre de kératine pendant le temps d'action est effectué visuellement sans enlever le mélange d'application de la fibre.

15. Utilisation d'hydrolysats de kératine pour augmenter le pouvoir éclaircissant et régénérant de l'agent de blondissement transparent.
